# EUROPEAN PATENT APPLICATION

(11) **EP 4 749 977 A2**
(43) Date of publication of application: **27.05.2026**
(21) Application number: 26161640.3
(22) Date of filing: 19.12.2022
(51) Int. Cl.: H04L 9/00

(54) **CHEMICAL PRODUCT PASSPORT**

(30) Priority: 21.12.2021 EP 21216268; 21.12.2021 EP 21216269; 21.12.2021 EP 21216270; 21.12.2021 EP 21216271; 21.12.2021 EP 21216286; 21.12.2021 EP 21216292; 21.12.2021 EP 21216326; 21.12.2021 EP 21216327; 21.12.2021 EP 21216333; 04.04.2022 EP 22166573; 12.04.2022 EP 22167945; 10.05.2022 EP 22172609; 10.05.2022 EP 22172611; 10.05.2022 EP 22172615; 10.05.2022 EP 22172617; 10.05.2022 EP 22172619; 09.09.2022 EP 22194793; 09.09.2022 EP 22194800; 09.09.2022 EP 22194808; 09.09.2022 EP 22194815; 09.09.2022 EP 22194818; 14.10.2022 EP 22201672; 14.10.2022 US 202263416091 P; 18.10.2022 EP 22202183; 05.12.2022 EP 22211421
(62) Divisional of application: 22835844.6
(71) Applicant: BASF SE, 67056 Ludwigshafen am Rhein (DE)
(72) Inventor: SCHWABE, Henning, 68163 Ludwigshafen am Rhein (DE); HAARDT, Dennis, 69469 Weinheim (DE); WOLLNY, Andreas, 67056 Ludwigshafen am Rhein (DE)
(74) Representative: BASF IP Association

(57) **Abstract**

Disclosed is an apparatus for generating a chemical product passport, the apparatus comprising:
one or more computing nodes; and one or more computer-readable media having thereon com puter-executable instructions that are structured such that, when executed by the one or more computing nodes, cause the apparatus to perform the following steps: receiving a request to provide a decentral identifier associated with emission data and a data owner,
in response to the request, generating the chemical product passport including the decentral identifier and data related to the emission data; providing the chemical product passport for access by a data consuming service controlled by a data providing service associated with the data owner.

## Description

### TECHNICAL FIELD

The present disclosure relates to an apparatus for generating a chemical product passport, a computer-implemented method for generating a chemical product passport, a method for using a chemical product passport and a computer program element.

### TECHNICAL BACKGROUND

In the supply of chemical products multiple regulatory requirements need to be met, which differ depending on the chemical product. For instance, in automotive supply chains chemical companies provide standardized information using the International Material Data System (IMDS). Such system allows to collect data along the entire automotive supply chain. Participants in the automotive supply chain register with the IMDS service and maintain product entries in the central database as provided and hosted by a third-party provider.

Systems like IMDS are static regarding data, prone to error and cumbersome in handling or maintenance. Owing to the highly specific and centralized setup of such systems, exchange and sharing of chemicals data is laborious. Hence there is a need to simplify chemical data exchange and sharing.

### SUMMARY OF THE INVENTION

In one aspect an apparatus for generating a chemical product passport is disclosed, the apparatus comprising: one or more computing nodes; and one or more computer-readable media having thereon computer-executable instructions that are structured such that, when executed by the one or more computing nodes, cause the apparatus to perform the following steps: receiving a request to provide a decentral identifier associated with a data owner and emission data,

in response to the request, generating the chemical product passport including the decentral identifier and data related to the emission data; providing the chemical product passport for access by a data consuming service under control or controlled by a data providing service associated with the data owner.

In one aspect an apparatus for generating a chemical product passport is disclosed, the apparatus comprising: one or more computing nodes; and one or more computer-readable media having thereon computer-executable instructions that are structured such that, when executed by the one or more computing nodes, cause the apparatus to perform the following steps: receiving a request to provide a decentral identifier associated with a data owner and emission data, in response to the request, providing the decentral identifier and generating the chemical product passport including the decentral identifier and data related to the emission data; providing the chemical product passport for access by a data consuming service under control or controlled by a data providing service associated with the data owner.

In one aspect an apparatus for generating a chemical product passport is disclosed, the apparatus comprising: one or more computing nodes; and one or more computer-readable media having thereon computer-executable instructions that are structured such that, when executed by the one or more computing nodes, cause the apparatus to perform the following steps:
providing a decentral identifier associated with a data owner and emission data, generating the chemical product passport including the decentral identifier and data related to the emission data;
providing the chemical product passport for access by a data consuming service controlled by a data providing service associated with a data owner.

In one aspect disclosed is an apparatus an apparatus for generating a chemical product passport including a decentral identifier and data related to emission data, particularly a digital representation of emission data, the apparatus comprising:
one or more computing nodes; and one or more computer-readable media having thereon computer-executable instructions that are structured such that, when executed by the one or more computing nodes, cause the apparatus to perform the following steps:
receiving a request to provide the decentral identifier associated with emission data and a data owner, particularly wherein the data owner controls access to the emission data, e.g. by a data consuming service;

in response to the request, providing the decentral identifier and generating the chemical product passport including the decentral identifier and the data related to the emission data; providing the chemical product passport for access by the data consuming service controlled by or under control by a data providing service associated with the data owner.

In one aspect disclosed is an apparatus for generating a chemical product passport including a decentral identifier and data related to emission data, particularly a digital representation of emission data, the apparatus comprising:
one or more computing nodes; and one or more computer-readable media having thereon computer-executable instructions that are structured such that, when executed by the one or more computing nodes, cause the apparatus to perform the following steps:
providing the decentral identifier associated with emission data and a data owner, particularly wherein the data owner controls access to the emission data, e.g. by a data consuming service;
generating the chemical product passport including the decentral identifier and the data related to the emission data;
providing the chemical product passport for access by the data consuming service controlled by or under control by a data providing service associated with the data owner.

In one aspect disclosed is an apparatus for generating a chemical product passport, particularly including the decentral identifier and the data related to the emission data, the apparatus comprising:
one or more computing nodes; and one or more computer-readable media having thereon computer-executable instructions that are structured such that, when executed by the one or more computing nodes, cause the apparatus to perform the following steps:
receiving a request to provide a decentral identifier associated with emission data and a data owner;
in response to the request, providing the decentral identifier and generating the chemical product passport including the decentral identifier and the data related to the emission data;
providing the chemical product passport for access by the data consuming service controlled by or under control by a data providing service associated with the data owner, particularly wherein the data providing service comprises computer-executable instructions for providing and/or processing emission data associated with the data owner e.g. for accessing and/or processing by the data consuming service.

In one aspect disclosed is an apparatus for generating a chemical product passport, particularly including the decentral identifier and the data related to the emission data, the apparatus comprising:
one or more computing nodes; and one or more computer-readable media having thereon computer-executable instructions that are structured such that, when executed by the one or more computing nodes, cause the apparatus to perform the following steps:
providing a decentral identifier associated with emission data and a data owner;

generating the chemical product passport including the decentral identifier and the data related to the emission data;
providing the chemical product passport for access by the data consuming service controlled by or under control by a data providing service associated with the data owner, particularly wherein the data providing service comprises computer-executable instructions for providing and/or processing emission data associated with the data owner e.g. for accessing and/or processing by the data consuming service.

In another aspect a computer-implemented method for generating a chemical product passport is disclosed, the method comprising the steps:
receiving a request to provide a decentral identifier associated with a data owner and emission data,

in response to the request, generating the chemical product passport including the decentral identifier and data related to the emission data;
providing the chemical product passport for access by a data consuming service controlled by a data providing service associated with the data owner.

In another aspect a computer-implemented method for generating a chemical product passport is disclosed, the method comprising the steps:
providing a decentral identifier associated with a data owner and emission data, generating the chemical product passport including the decentral identifier and data related to the emission data;
providing the chemical product passport for access by a data consuming service controlled by a data providing service associated with the data owner.

In another aspect a computer-implemented method for generating a chemical product passport including a decentral identifier and data related to emission data, particularly a digital representation of emission data is disclosed, the method comprising the steps:
receiving a request to provide the decentral identifier associated with emission data and a data owner, particularly wherein the data owner controls access to the emission data, e.g. by a data consuming service;

in response to the request, providing the decentral identifier and generating the chemical product passport including the decentral identifier and the data related to the emission data; providing the chemical product passport for access by the data consuming service controlled by or under control by a data providing service associated with the data owner.

In another aspect a computer-implemented method for generating a chemical product passport including a decentral identifier and data related to emission data, particularly a digital representation of emission data is disclosed, the method comprising the steps:
providing the decentral identifier associated with emission data and a data owner, particularly wherein the data owner controls access to the emission data, e.g. by a data consuming service; generating the chemical product passport including the decentral identifier and the data related to the emission data;
providing the chemical product passport for access by the data consuming service controlled by or under control by a data providing service associated with the data owner.

In another aspect a computer-implemented method for generating a chemical product passport including a decentral identifier and data related to emission data, particularly a digital representation of emission data is disclosed, the method comprising the steps:
receiving a request to provide a decentral identifier associated with emission data and a data owner;
in response to the request, providing the decentral identifier and generating the chemical product passport including the decentral identifier and the data related to the emission data;
providing the chemical product passport for access by the data consuming service controlled by or under control by a data providing service associated with the data owner, particularly wherein the data providing service comprises computer-executable instructions for providing and/or processing emission data associated with the data owner e.g. for accessing and/or processing by the data consuming service.

In another aspect a computer-implemented method for generating a chemical product passport including a decentral identifier and data related to emission data, particularly a digital representation of emission data is disclosed, the method comprising the steps:
providing a decentral identifier associated with emission data and a data owner;
generating the chemical product passport including the decentral identifier and the data related to the emission data;
providing the chemical product passport for access by the data consuming service controlled by or under control by a data providing service associated with the data owner, particularly wherein the data providing service comprises computer-executable instructions for providing and/or processing emission data associated with the data owner e.g. for accessing and/or processing by the data consuming service.

In yet another aspect a computer-implemented method for using a chemical product passport, preferably to further process the chemical product associated with the chemical product passport, is disclosed, the method comprising the steps:
receiving a request to access the emission data associated with a decentral identifier of the chemical product passport as generated according to the methods disclosed herein or by the apparatuses disclosed herein;

optionally authenticating and/or authorizing the request to access the emission data;
based on optionally the authentication and/or authorization, providing access to emission data associated with the decentral identifier of the chemical product passport.

Use of the chemical product passport generated for the chemical product according to the methods or by the apparatuses lined out herein to further process the chemical product associated with the chemical product passport.

In yet another aspect a chemical product associated with the chemical product passport is disclosed, wherein the chemical product passport including the decentral identifier and data related to the emission data is generated for the chemical product according to the methods or by the apparatuses lined out herein.

In yet another aspect a system including a chemical product associated with the chemical product passport is disclosed, wherein the chemical product passport including the decentral identifier and data related to the emission data is generated for the chemical product according to the methods or by the apparatuses lined out herein.

In yet another aspect a chemical product passport including the decentral identifier and data related to the emission data is disclosed, wherein the chemical product passport is generated for the chemical product according to the methods or by the apparatuses lined out herein.

In yet another aspect a computer element, in particular a computer program product or a computer readable medium, with instructions, which when executed on one or more computing node(s) is configured to carry out the steps of any of the methods or by the apparatus disclosed herein is disclosed.

Any disclosure and embodiments described herein relate to the methods, the apparatuses, the systems, the chemical products, the chemical product passports, the uses, the computer element lined out above or below and vice versa. The benefits provided by any of the embodiments and examples equally apply to all other embodiments and examples.

As used herein "determining" also includes "initiating or causing to determine", "generating" also includes "initiating and/or causing to generate" and "providing" also includes "initiating or causing to determine, generate, select, send and/or receive". "Initiating or causing to perform an action" includes any processing signal that triggers a computing node or device to perform the respective action.

The methods, apparatuses and computer elements disclosed herein provide an efficient, secure and robust way for sharing or exchanging data across different participant nodes in chemical value chains. In particular, a) by attaching a decentral identifier to a data owner and associated emission data and b) by providing access by a data consuming service controlled by a data providing service associated with the data owner, data can be securely exchanged and shared under the sovereignty of the data owner. The data owner may thus control access by participant nodes or data consuming services of the decentral network to the chemical product data. This allows for simplified and customizable data sharing or exchange from chemical industry to chemical supply chain participants. This way, a more reliable and efficient further processing of supplied chemical product by upstream participants of the chemical supply chain can be achieved, while the data remains in the ownership of the chemical supplier supplying the upstream participant. By combining the data related to chemicals directly with the decentral identifier and optionally one or more authentication mechanisms more reliable and secure data sharing and exchange can be provided. By further including one or more authorization mechanisms, the data sharing or exchange can be conducted in a more flexible manner with multiple data consuming services from different participants of the chemical supply chain accessing the emission data.

The term "decentral identifier" is to be understood broadly in the present case. The decentral identifier may comprise any unique identifier uniquely associated with the data owner and chemical product data, in particular the emission data. The decentral identifier may include one or more Universally Unique Identifier(s) (UUID) or a Digital Identifier(s) (DID). The decentral identifier may be issued by a central or decentral identity issuer. The decentral identifier may include authentication information. Via the decentral identifier and its unique association with the data owner and chemical product data, in particular emission data, access to the chemical product data, in particular emission data, may be controlled by the data owner. This contrasts with central authority schemes, where identifiers are provided by such central authority and access to data is controlled by such central authority. Decentral in this context refers to the usage of the identifier in implementation as controlled by the data owner.

The decentral identifier may include one or more identifier(s) used in the decentral network and allowing for data exchange via the decentral network. Data exchange may include discovery of the decentral identifier for participant nodes of the decentral network, authentication of participant nodes of the decentral network and/or authorization of data transfers via a peer-to-peer communication between participant nodes of the decentral network. The decentral identifier may be associated with any participant of the supply chain including raw chemical product supplier, intermediate chemical products manufacturer, intermediate part manufacturer, component manufacturer, component assembly manufacturer or end product manufacturer. The decentral identifier may be associated with a machine, a system, or a device used for producing the raw material, the basic substance, the chemical product, the intermediate product, the component, the compo-nent assembly or the end product, or a collection of such machine(s), device(s) and/or system(s).

The term "chemical product passport" is to be understood broadly in the present case. The chemical product passport may include data related to the chemical product. The chemical product passport may include a digital representation of the chemical product data associated with the chemical product. The chemical product passport may comprise or be associated with a digital representation of chemical product data, in particular emission data. The chemical product data may be associated with the chemical product. The digital representation of the chemical product data may be provided to the data consuming service. The digital representation of the chemical product data may be provided by a decentral network database, a database associated with the data consuming, the data providing service associated with the data owner or combinations thereof. The digital representation may include a representation for accessing the chemical product data, in particular emission data, or part thereof. The digital representation may include a representation of chemical product data, in particular emission data, or parts thereof. The chemical product passport may include data related to the chemical product data, in particular emission data, the public key and the decentral identifier. The data related to the chemical product data, in particular emission data, may include the digital representation of the chemical product data, in particular emission data.

The term "chemical product" is to be understood broadly in the present case. The chemical product may comprise chemical products obtained from chemical reactions as well as natural chemical products. Natural chemical products encompass any chemical substance that is naturally occurring, i.e. any unprocessed chemical substance that is found in nature, such as chemicals from plants, micro-organisms, animals, the earth and the sea or any chemical substance that is found in nature and extracted using a process that does not change its chemical composition. Natural chemical products may include biologicals like enzymes as well naturally occurring inorganic or organic chemical products. Natural chemical products can be isolated and purified prior to their use or they can be used in unisolated and/or unpurified form. Chemical products obtained from chemical reactions may be any inorganic or organic chemical product obtained by reacting inorganic and/or organic chemical reactants. The inorganic and organic chemical reactants may be naturally occurring chemical products or can be chemical products obtained from chemical reactions. Chemical reactions may include any chemical reaction commonly known in the state of the art in which the reactants are converted to one or more different chemical products. Chemical reactions may involve the use of catalysts, enzymes, bacteria, etc. to achieve the chemical reaction between the reactants.

The term "chemical product data" is to be understood broadly in the present case. The chemical product data may be associated with the chemical product. The chemical product data may comprise data related to a property of the chemical product and/or data related to the use of the chemical product. Such property may be a static or a dynamic property. A static property may be a property constant over time e.g. melting point, boiling point, density, hardness, flammability our the like. A dynamic property may be a property that changes over time e.g. shelf life, **pH** value, color, reactivity. The property of the chemical product may include performance properties, chemical properties, such as flammability, toxicity, acidity, reactivity, heat of combustion and/or physical properties such as density, color, hardness, melting and boiling points, electrical conductivity or the like. Data related to the use of the chemical product may include data related to further processing of the chemical product, for example by using the chemical product as reactant in further chemical reaction(s) and/or data related to the use of the chemical product, for example data related to the use of the chemical product in a treatment process and/or within a manufacturing process. Chemical product data may include chemicals data, emission data, recyclate content, bio-based content and/or production data.

The term "physical entity" is to be understood broadly in the present case. The physical entity may relate to the physical embodiment of a product. The physical entity may be any prodcut in the chemical supply chain. The physical entity of a product may be a raw material or basic substance, a chemical product, a chemical material, a chemical formulation, a chemical mixture, a component, a component assembly, an end product or a combination thereof.

The term "recyclate content data and/or bio-based content data" is to be understood broadly in the present case. Recyclate content data and/or bio-based content data maycomprise any data related to the recyclate content or the bio-based content used for providing or manufacturing a physical entity or product at any stage in the chemical supply chain such as a raw material or basic substance, chemical product or chemical material, component, component assembly or end product.

The term "emission data" is to be understood broadly in the present case. The emission data may comprise any data related to environmental footprint. The environmental footprint may refer to an entity and its associated environmental footprint. The environmental footprint may be entity specific. For instance, the environmental footprint may relate to a product, a company, a process such as a manufacturing process, a raw material or basic substance, a chemical product or material, a component, a component assembly, an end product, combinations thereof or additional entity-specific relations. Emission data may include data relating to the carbon footprint of the chemical product or a Product Carbon Footprint (PCF). Emission data may include data relating to greenhouse gas emissions e.g. released in production of the chemical product. Emission data may include data related to greenhouse gas emissions. Greenhouse gas emissions may include emissions such as carbon dioxide (CO2) emission, methane (CH4) emission, nitrous oxide (N2O) emission, hydrofluorocarbons (HFCs) emission, perfluorocarbons (PFCs) emission, sulphurhexafluoride (SF6) emission, nitrogen trifluoride (NF3) emission, combinations thereof and additional emissions.

Emission data may include data related to greenhouse gas emissions of an entities or companies own operations (production, power plants and waste incineration). Scope 2 may comprise emissions from energy production which is sourced externally. Scope 3 may comprise all other emissions along the value chain. Specifically, this may include the greenhouse gas emissions of raw materials obtained from suppliers. Product Carbon Footprint (PCF) may sum up greenhouse gas emissions and removals from the consecutive and interlinked process steps related to a particular product. Cradle-to-gate PCF may sum up greenhouse gas emissions based on selected process steps: from the extraction of resources up to the factory gate where the product leaves the company. Such PCFs may be called partial PCFs. In order to achieve such summation, each company providing any products may provide the scope 1 and scope 2 contributions to the PCF for each of its products.

The term "production data" is to be understood broadly in the present case. The production data may comprise any data related to the production of a product at any stage in the chemical supply chain. Preferably production data includes chemical production data from the production of the chemical product. Production data may include monitoring and/or control data associated with the production of the product, such as a raw material or basic substance, a chemical material or chemical product, a component, a component assembly, an end product or a combination thereof. Production data may include measurement data related to a product quality at any stage in the chemical supply chain, preferably the chemical product.

The term "data owner" is to be understood broadly in the present case. The data owner comprise any entity generating data, particularly chemical product data or emission data. The generating node may be coupled to the entity owning physical products from or for which data, particularly chemical product data or emission data, is generated. The data may be generated by a third-party entity on behalf of the entity owning physical products from or for which data is generated. Via the decentral identifier and its unique association with the data owner and chemical product data access to the chemical product data may be controlled by the data owner. The chemical product data may be accessible for the data owner. The data owner may hence directly or indirectly own the chemical product data. The chemical product data may be stored in a data base of or associated with the data owner. The chemical product data may be stored in a data base accessible by the data owner. The data owner may control access to the chemical product data via the data providing service of the data owner. The data owner may control access to the chemical product data. The chemical product data may be associated with the data owner. The data owner may be the owner of the chemical product data or the chemical product data owner. The chemical product data may be stored in a data base of or under control by the data owner. In this sense, the data owner is to be construed broadly as the entity having access to the chemical product data and controlling access by data consuming services of the decentral network to the chemical product data. The data owner may be the chemical product producer.

The term "data consuming service" is to be understood broadly in the present case- The data consuming service may comprise computer-executable instructions for accessing and/or processing data, such as chemical product data or emission data, associated with the data owner.

The term "data providing service" is to be understood broadly in the present case. The data providing service may comprise computer-executable instructions for providing and/or processing data, such as chemical product data or emission data, associated with the data owner for accessing and/or processing by a data consuming service.

The term "digital representation(s) pointing to product data or parts thereof" is to be understood broadly in the present case. The digital representation(s) pointing to product data or parts thereofmay comprise at least one interface to the data providing service. It may further include at least one interface to the data consuming service. It may include an endpoint for data exchange or sharing (resource endpoint) or an endpoint for service interaction (service Endpoint), that is uniquely identified via a communication protocol. The digital representation(s) pointing to product data or parts thereof may hence be uniquely associated with the decentral identifier.

In one embodiment the request to provide the decentral identifier includes data related to the chemical product data, in particular emission data, and an owner identifier associated with the chemical product data owner, in particular emission data owner. The owner identifier may be a string identifier associated with a data owner name. The owner or product identifier may be provided by a physical identifier provider, such as a bar code or a tag like a RFID tag or QR code. Such communication can also be completed via ad hoc WIFI, BLE beacon, and/or NFC. The communications between the wallet apps may be performed via any available communication channel, including but not limited to, web servers, ad hoc WIFI, BLE beacon signal, NFC, a bar-code or QR code scanning, etc.

Through the owner identifier the chemical product passport generated may be associated with the chemical product data owner or in particular emission data owner by including the owner identifier. The owner identifier may be used for data transaction, such as sharing or exchanging chemical product data, in particular emission data. The owner identifier may be provided to a transaction manager. By providing the decentral identifier and the owner identifier of the data owner to a transaction manager or a data consuming service may simplify tracking of data transactions. Any transaction in the data ecosystem can e.g. be associated with the clear name of the data owner.

In one embodiment the decentral identifier is provided by one central node or by one or more decentral nodes. The decentral identifier as generated by one central node or by one or more decentral nodes may be provided to a node generating the chemical product passport and to at least one authentication data registry node, preferably accessible by the data providing service and/or the data consuming service. This enables customized data sharing or exchange with respect to the chemical product and the chemical supply chain the chemical product is supplied to. In particular, the data providing service and/or the data consuming service may customize data sharing or exchange protocols based on the anchoring of the decentral identifier to chemical product data, in particular emission data.

The authentication data registry node may be a central registry node such as a central file system, a centrally managed distributed database, and/or a centrally managed peer-to-peer network. The central configuration allows for more control and standardization via a central node. The authentication data registry node may be a decentral registry such as a distributed ledger, a decentralized file system, a distributed database, and/or a peer-to-peer network. The decentral configuration allows for more efficient use of computing resources and strengthens control by the data owner. In addition, the decentral configuration is independent from centrally managed nodes and as such increases reliability and flexibility of the system.

In one embodiment the generation of the chemical product passport includes providing the decentral identifier associated with a physical entity of a product. In this context the physical entity may relate to a physical product that is associated with the decentral identifier. The physical entity may be any entity in the chemical supply chain. It may relate to raw materials such as crude oil, chemical products such as polyols and diisocyanates or polyurethan intermediates produced from polyols and diisocyanates, components, such as foamed parts or beats, component assemblies such as car seats or shoe soles and/or end products such as cars.

The decentral identifier may be associated with the physical entity the chemical product data, in particular emission data, is associated with. The decentral identifier may be associated with the chemical product the chemical product data, in particular emission data, is associated with. For instance, the decentral identifier may be associated with the physical entity of the basic substance, the raw material, the chemical material, the chemical mixture, the chemical formulation or the like. The decentral identifier may be associated with the physical entity the chemical product will be supplied for and the chemical product data, in particular emission data, is associated with. For instance, the decentral identifier may be associated with the physical entity of the component, the component assembly, the end product or the like. The decentral identifier may be associated with more than one physical entity the chemical product will be supplied for and the chemical product data, in particular emission data, is associated with. For instance, the decentral identifier may be associated with the physical entity of the component, the component assembly and the end product. Associating the decentral identifier with different physical entity stages in the chemical supply chain allows for virtually tracking the supplied chemical product in the supply chain. This way the chemical product with its associated chemical product data, in particular emission data, may be tracked e.g. up to the end of life of the end product.

In one embodiment the chemical product passport includes one or more authentication mechanisms associated with the decentral identifier and the data related to the chemical product data, in particular emission data. The authentication mechanism may directly or indirectly relate to the decentral identifier and the data related to the chemical product data. In an example of indirect relation, the authentication mechanism may relate to a certificate mechanism. For example, on access request by the data consuming service, a dynamic access token may be generated based on the certificate mechanism. Such dynamic access token may be used to open peer-to-peer communication channel between data consuming service and the data providing service. The authentication mechanism may include a token, such as private and public key infrastructure, a certificate mechanism or a biometric mechanism, such as fingerprints, face recognition or voice recognition or the like. One common public key certificate is for instance the X.509 certificate. Through the authentication mechanism data access by a data consuming service can be controlled in a secure manner and integrity of the data providing service can be ensured. This allows for more reliable, controlled and secure data exchange or sharing.

The one or more authentication mechanisms associated with the decentral identifier as generated by one central node or by one or more decentral nodes may be provided to a node generating the chemical product passport and to at least one decentral authentication data registry, preferably accessible by the data providing service and/or the data consuming service. The authentication data registry may be a central registry such as a central file system, a centrally managed distributed database, and/or a centrally managed peer-to-peer network. The central configuration allows for higher control and standardization via a central node. The authentication data registry may be a decentral registry such as a distributed ledger, a decentralized file system, a distributed database, and/or a peer-to-peer network. The decentral configuration allows for more efficient use of computing resources and strengthens control by the data owner. In one embodiment the chemical product passport is related to or includes one or more authorization mechanisms associated with the decentral identifier and the data related to the chemical product data, in particular emission data. The authorization mechanisms may include authorization rule(s) including data transaction instructions or data transaction protocols, such as data usage policies, smart data contracts or mor complex data processing instructions associated with data providing and/or data consuming services. Through the authorization mechanism data access and data usage by a data consuming service can be controlled in a secure manner.

The one or more authorization mechanisms associated with the decentral identifier as generated by one central node or by one or more decentral nodes may be provided to a node for generating or processing the chemical product passport or for accessing the data related to the chemical product data, in particular emission data. Additionally or alternatively, the one or more authorization mechanisms may be provided to at least one central or decentral authorization data registry, preferably accessible by the data providing service and/or the data consuming service.

In one embodiment the one or more authorization mechanisms associated with the decentral identifier as generated by one or more decentral nodes may be provided to a node generating or processing the chemical product passport and to at least one of a central file system, a centrally managed distributed database, a centrally managed peer-to-peer network, a distributed ledger, a decentralized file system, a distributed database, and/or a peer-to-peer network, preferably accessible by the data providing service and/or the data consuming service.

In one embodiment the data related to chemical product data, in particular emission data, includes chemical product data, in particular emission data, or parts thereof. In one embodiment the data related to chemical product data, in particular emission data, includes one or more digital representation(s) pointing to chemical product data, in particular emission data, or parts thereof. In this context pointing means any network representation or address that is suitable for accessing the chemical product data, in particular emission data. The data related to chemical product data, in particular emission data, may include multiple digital representations pointing to distinct parts of the chemical product data, in particular emission data. The data related to chemical product data, in particular emission data, may include multiple digital representations pointing to different parts of the chemical product data, in particular emission data. Such different parts may overlap in some data points. The representation may include an access point to the chemical product data, in particular emission data, a link to access chemical product data, in particular emission data, an endpoint to access chemical product data, in particular emission data, or a service endpoint to access chemical product data, in particular emission data. This way the chemical product data, in particular emission data, can be maintained and controlled by a data owner. Access can be provided via the representation of an access point simplifying data verification, integrity checks or quality checks and access control, since not multiple distributed data points need to be checked and access controlled. The chemical product data, in particular the emission data may be stored in a data base of or associated with the data owner. The chemical product data, in particular the emission data may be stored in a data base accessible by the data owner. The digital representation pointing to chemical product data, in particular the emission data, or parts thereof may be associated with or relate to any such data base associated with or accessible by the data owner. For enhanced security the digital representation pointing to chemical product data, in particular emission data or parts thereof may indirectly relate to any such data base associated with or accessible by the data owner.

In one embodiment the chemical product passport includes data related to is associated with one or more class(es) of chemical product data, in particular emission data. For instance, the data related to chemical product data, in particular emission data, includes one or more digital representations pointing to different classes of the chemical product data, in particular emission data. In one embodiment the chemical product passport includes data related to different class(es) of chemical product data, in particular emission data. For instance, the data related to chemical product data includes multiple digital representations pointing to different classes of the chemical product data, in particular emission data.

In one embodiment art least one class of chemical product data is associated with or includes a representation of chemicals data e.g. data required by regulation or regulatory data for chemicals. The chemical product passport may include data related to chemicals data. Chemicals data may include chemical product declaration data associated with the physical entity of the chemical product the product made from the chemical product or the product, such as the raw material, the chemical product, the component, the component assembly, or the end product and/or combinations thereof. The chemical product declaration data may by associated with more than one raw material or chemical product such as those used to manufacture the component. The chemical product declaration data may by associated with more than one raw material or chemical product such as those used to manufacture multiple components to assemble component assembly or end product.

In one embodiment chemicals data include chemical product safety data associated with hazards of substances or mixtures of the physical entity of the product such as the raw material, the chemical product, the component, the component assembly, or the end product and/or combinations thereof. The chemical product safety data may by associated with more than one raw material or chemical product such as those used to manufacture the component. The chemical product safety data may by associated with more than one raw material or chemical product such as those used to manufacture multiple components to assemble component assembly or end product.

In one embodiment chemicals data include certificate of analysis data associated with laboratory measurement data acquired from a sample of the raw material, the chemical product, the component, the component assembly, or the end product and/or combinations thereof. The certificate of analysis data may by associated with more than one raw material or chemical product such as those used to manufacture the component. The certificate of analysis data may by associated with more than one raw material or chemical product such as those used to manufacture multiple components to assemble component assembly or end product.

In one embodiment at least one class of chemical product data include emission data, recyclate content data, bio-based content data and/or production data associated with the physical entity of the product such as the raw material, the chemical product, the component, the component assembly, the end product and/or combinations thereof. The chemical product passport may include data related to emission data, recyclate content data, bio-based content data and/or production data. The emission data, recyclate content data, bio-based content data and/or production data may be associated with more than one raw material or chemical product such as those used to manufacture the component. The emission data, recyclate content data, bio-based content data and/or production data may be associated with more than one raw material or chemical product such as those used to manufacture multiple components to assemble a component assembly or an end product.

In one embodiment at least one class of chemical product data, in particular emission data, is associated with or includes access restricted chemical product data, in particular emission data, associated with the physical entity of the product such as the raw material, the chemical product, the component, the component assembly, the end product and/or combinations thereof. For instance, emission data, recyclate content data, bio-based content data, production data, or combinations thereof may be access restricted. Such access restriction may be provided by an authorization mechanism. For instance, the authorization mechanism may include a rule that specifies which data consuming services get access under which conditions.

In one embodiment at least one class of chemical product data include non-access restricted chemical product data associated with the physical entity of the product such as the raw material, the chemical product, the component, the component assembly, the end product and/or combinations thereof. For instance, chemical product declaration data, chemical product safety data, and/or certificate of analysis data associated with the physical entity of the product such as the raw material, the chemical product, the component, the component assembly, or the end product and/or combinations thereof may not be access restricted or non-access restricted. Such access may be provided by an authorization mechanism. For instance, the authorization mechanism may include a rule that specifies that certain regulatory data for chemicals is accessible.

### BRIEF DESCRIPTION OF THE DRAWINGS

In the following, the present disclosure is further described with reference to the enclosed figures:
- Figs. 1a,b: illustrate example embodiments of a centralized and a decentralized computing environment with computing nodes.
- Figs. 1c: illustrate an example embodiment of a distributed computing environment.
- Figure 2: shows an example of a chemical product passport including DID owner data, DID document data and decentral identity infrastructure.
- Figure 3: shows an example of a chemical product passport including ID-based data, passport data and decentral identity infrastructure.
- Figure 4: shows an example method for generation of the chemical product passport.
- Figure 5: shows an example method for using the chemical product passport to further process the chemical product associated with the chemical product passport.
- Figs. 6a, 6b: show examples of authentication protocols.
- Figs. 7a, 7b: illustrate the principles of cryptographic signatures.
- Figure 8: shows an example method for authorizing access to chemical product data, in particular emission data.
- Figure 9: shows a schematic illustration of providing access to product passport of a data providing service in the International Data Space (IDS) architecture to a data consuming service.
- Figure 10: shows a schematic illustration of the authentication process between two IDS connectors.
- Figure 11: shows a schematic illustration of a usage-controlled data flow in the IDS architecture.
- Fig. 12: illustrates an example of an apparatus and associated methods for generating the product passport in connection with a chemical product produced by a chemical production network.
- Figs. 13-15: show different example configurations for product passports anchored by digital identifiers.

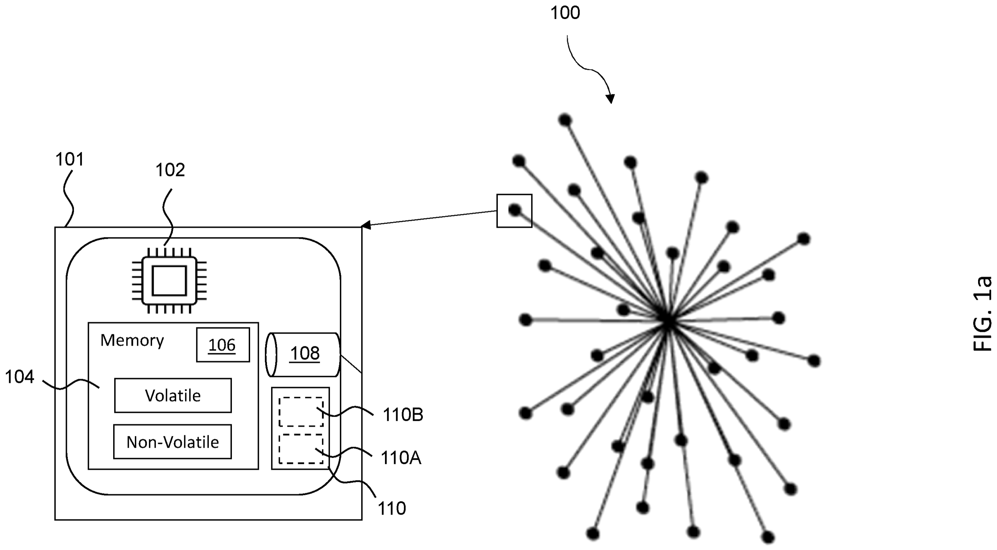

Figs. 1a to 1c illustrate different computing environments, central, decentral and distributed. The methods, apparatuses, systems, passports, computer elements of this disclosure may be implemented in decentral or at least partially decentral computing environments. In particular, for data sharing or exchange in ecosystems of multiple players different challenges exist. Data sovereignty may be viewed as a core challenge. It can be defined as a natural person's or corporate entity's capability of being entirely self-determined with regard to its data. To enable this particular capability related aspects, including requirements for secure and trusted data exchange in business ecosystems, may be implemented across the chemical value chain. In particular, chemical industry requires tailored solutions to deliver chemical products in a more sustainable way by using digital ecosystems.

Figure 1a illustrates an example embodiment of a centralized computing system 100 comprising a central computing node 101 (filled circle in the middle) and several peripheral computing nodes 101.1 to 101.n (denoted as filled circles in the periphery). The term "computing system" may include one or more computing nodes, a system of nodes or combinations thereof. The term "computing node" may refer to any device or system that includes at least one physical and tangible processor, and a physical and tangible memory capable of having thereon computer-executable instructions that are executed by a processor. Computing nodes are now increasingly taking a wide variety of forms. Computing nodes may, for example, be handheld devices, production facilities, sensors, monitoring systems, control systems, appliances, laptop computers, desktop computers, mainframes, data centers, or even devices that have not conventionally been considered a computing node, such as wearables (e.g., glasses, watches or the like). The memory may take any form and may depend on the nature and form of the computing node.

In this example, the peripheral computing nodes 101.1 to 101.n may be connected to one central computing system (or server). In another example, the peripheral computing nodes 101.1 to 101.n may be attached to the central computing node via e.g. a terminal server (not shown).

The majority of functions may be carried out by, or obtained from the central computing node (also called remote centralized location). One peripheral computing node 101.n has been expanded to provide an overview of the components present in the peripheral computing node. The central computing node 101 may comprise the same components as described in relation to the peripheral computing node 101.n.

Each computing node 101, 101.1 to 101.n may include at least one hardware processor 102 and memory 104. The term "processor" may refer to an arbitrary logic circuitry configured to perform basic operations of a computer or system, and/or, generally, to a device which is configured for performing calculations or logic operations. In particular, the processor, or computer processor may be configured for processing basic instructions that drive the computer or system. It may be a semi-conductor based processor, a quantum processor, or any other type of processor configures for processing instructions. As an example, the processor may comprise at least one arithmetic logic unit ("ALU"), at least one floating-point unit ("FPU)", such as a math coprocessor or a numeric coprocessor, a plurality of registers, specifically registers configured for supplying operands to the ALU and storing results of operations, and a memory, such as an L1 and L2 cache memory. In particular, the processor may be a multicore processor. Specifically, the processor may be or may comprise a Central Processing Unit ("CPU"). The processor may be a ("GPU") graphics processing unit, ("TPU") tensor processing unit, ("CISC") Complex Instruction Set Computing microprocessor, Reduced Instruction Set Computing ("RISC") microprocessor, Very Long Instruction Word ("VLIW") microprocessor, or a processor implementing other instruction sets or processors implementing a combination of instruction sets. The processing means may also be one or more special-purpose processing devices such as an Application-Specific Integrated Circuit ("ASIC"), a Field Programmable Gate Array ("FPGA"), a Complex Programmable Logic Device ("CPLD"), a Digital Signal Processor ("DSP"), a network processor, or the like. The methods, apparatuses, systems and devices described herein may be implemented as software in a DSP, in a micro-controller, or in any other side-processor or as hardware circuit within an ASIC, CPLD, or FPGA. It is to be understood that the term processor may also refer to one or more processing devices, such as a distributed system of processing devices located across multiple computer systems (e.g., cloud computing), and is not limited to a single device unless otherwise specified.

The memory 104 may refer to a physical system memory, which may be volatile, non-volatile, or a combination thereof. The memory may include non-volatile mass storage such as physical storage media. The memory may be a computer-readable storage media such as RAM, ROM, EEPROM, CD-ROM, or other optical disk storage, magnetic disk storage, or other magnetic storage devices, or any other physical and tangible storage medium which can be used to store desired program code means in the form of computer-executable instructions or data structures and which can be accessed by the computing system. Moreover, the memory may be a computer-readable media that carries computer- executable instructions (also called transmission media). Further, upon reaching various computing system components, program code means in the form of computer-executable instructions or data structures can be transferred automatically from transmission media to storage media (or vice versa). For example, computer-executable instructions or data structures received over a network or data link can be buffered in **RAM** within a network interface module (e.g., a "NIC"), and then eventually transferred to computing system RAM and/or to less volatile storage media at a computing system. Thus, it should be understood that storage media can be included in computing components that also (or even primarily) utilize transmission media.

The computing nodes 101, 101.1...101.n may include multiple structures 106 often referred to as an "executable component or computer-executable instructions". For instance, memory 104 of the computing nodes 101, 101.1...101.n may be illustrated as including executable component 106. The term "executable component" may be the name for a structure that is well understood to one of ordinary skill in the art in the field of computing as being a structure that can be software, hardware, or a combination thereof or which can be implemented in software, hardware, or a combination. For instance, when implemented in software, one of ordinary skill in the art would understand that the structure of an executable component include software objects, routines, methods, and so forth, that is executed on the computing nodes 101, 101.1...101.n, whether such an executable component exists in the heap of a computing node 101, 101.1...101.n, or whether the executable component exists on computer-readable storage media. In such a case, one of ordinary skill in the art will recognize that the structure of the executable component exists on a computer-readable medium such that, when interpreted by one or more processors of a computing node 101, 101.1...101.n (e.g., by a processor thread), the computing node 101, 101.1...101n is caused to perform a function. Such a structure may be computer-readable directly by the processors (as is the case if the executable component were binary). Alternatively, the structure may be structured to be interpretable and/or compiled (whether in a single stage or in multiple stages) so as to generate such binary that is directly interpretable by the processors. Such an understanding of example structures of an executable component is well within the understanding of one of ordinary skill in the art of computing when using the term "executable component". Examples of executable components implemented in hardware include hardcoded or hard-wired logic gates, that are implemented exclusively or near-exclusively in hardware, such as within a field- programmable gate array (FPGA), an application-specific integrated circuit (ASIC), or any other specialized circuit. In this description, the terms "component", "agent", "manager", "service", "engine", "module", "virtual machine" or the like are used synonymous with the term "executable component.

The processor 102 of each computing node 101, 101.1...101.n may direct the operation of each computing node 101, 101.1...101.n in response to having executed computer- executable instructions that constitute an executable component. For example, such computer-executable instructions may be embodied on one or more computer-readable media that form a computer program product. The computer-executable instructions may be stored in the memory 104 of each computing node 101, 101.1...101.n. Computer-executable instructions comprise, for example, instructions and data which, when executed at a processor 101, cause a general purpose computing node 101, 101.1...101.n, special purpose computing node 101, 101.1...101.n, or special purpose processing device to perform a certain function or group of functions. Alternatively or in addition, the computer-executable instructions may configure the computing node 101, 101.1...101.n to perform a certain function or group of functions. The computer executable instructions may be, for example, binaries or even instructions that undergo some translation (such as compilation) before direct execution by the processors, such as intermediate format instructions such as assembly language, or even source code.

Each computing node 101, 101.1...101.n may contain communication channels 108 that allow each computing node 101.1...101.n to communicate with the central computing node 101, for example, a network (depicted as solid line between peripheral computing nodes and the central computing node in Fig. 1a). A "network" may be defined as one or more data links that enable the transport of electronic data between computing nodes 101, 101.1...101.n and/or modules and/or other electronic devices. When information is transferred or provided over a network or another communications connection (either hardwired, wireless, or a combination of hardwired or wireless) to a computing node 101, 101.1...101.n, the computing node 101, 101.1...101.n may view the connection as a transmission medium. Transmission media can include the network and/or data links which can be used to carry desired program code means in the form of computer-executable instructions or data structures and which can be accessed by a general-purpose or special-purpose computing nodes 101, 101.1...101.n. Combinations of the above may also be included within the scope of computer-readable media.

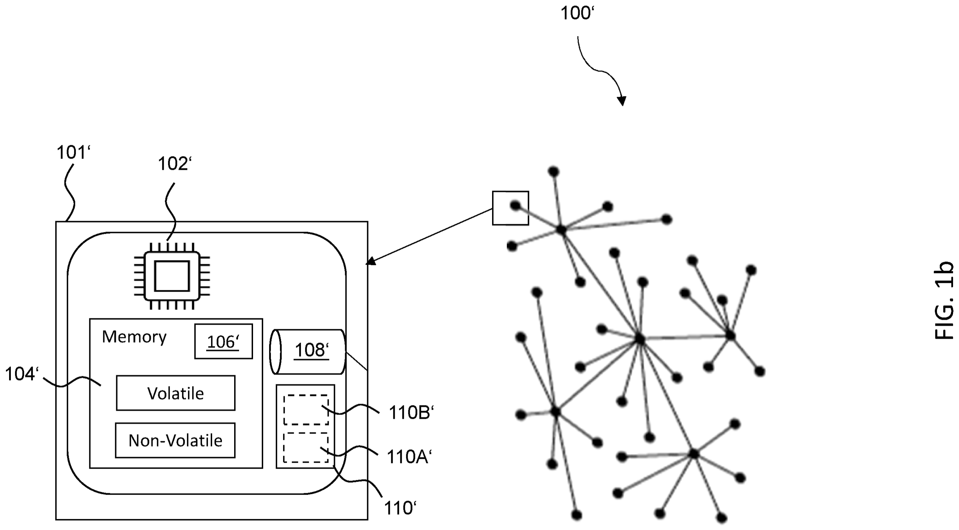

The computing node(s) 101, 101.1 to 101.n may further comprise a user interface system 110 for use in interfacing with a user. The user interface system 110 may include output mechanisms 110A as well as input mechanisms 110B. The principles described herein are not limited to the precise output mechanisms 110A or input mechanisms 110B as such will depend on the nature of the device. However, output mechanisms 110A might include, for instance, displays, speakers, displays, tactile output, holograms and so forth. Examples of input mechanisms 110B might include, for instance, microphones, touchscreens, holograms, cameras, keyboards, mouse or other pointer input, sensors of any type, and so forth. Figure 1b illustrates an example embodiment of a decentralized computing environment 100' with several computing nodes 101.1' to 101.n' denoted as filled circles. In contrast to the centralized computing environment 100 illustrated in Fig. 1a, the computing nodes 101.1' to 101.n' of the decentralized computing environment are not connected to a central computing node 101 and are thus not under control of a central computing node. Instead, resources, both hardware and software, may be allocated to each individual computing node 101.1'...101.n' (local or remote computing system) and data may be distributed among various computing nodes 101.1'...101.n' to perform the tasks. Thus, in a decentral system environment, program modules may be located in both local and remote memory storage devices. One computing node 101' has been expanded to provide an overview of the components present in the computing node 101'. In this example, the computing node 101' comprises the same components as described in relation to Fig. 1a.

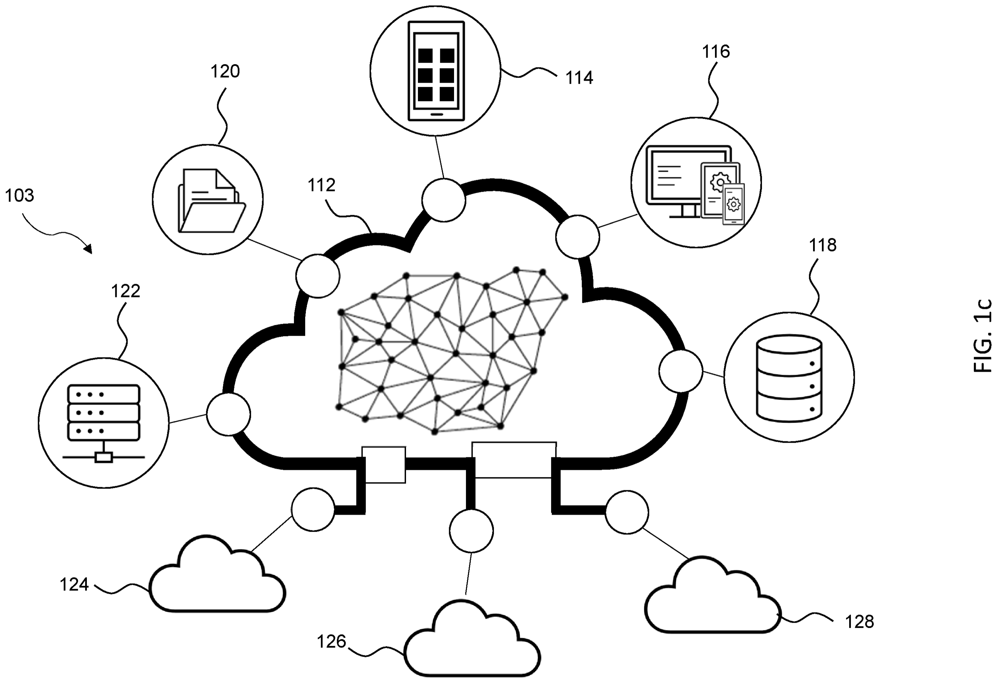

Figure 1c illustrates an example embodiment of a distributed computing environment 103. In this description, "distributed computing" may refer to any computing that utilizes multiple computing resources. Such use may be realized through virtualization of physical computing resources. One example of distributed computing is cloud computing. "Cloud computing" may refer a model for enabling on-demand network access to a shared pool of configurable computing resources (e.g., networks, servers, storage, applications, and services). When distributed, cloud computing environments may be distributed internationally within an organization and/or across multiple organizations. In this example, the distributed cloud computing environment 103 may contain the following computing resources: mobile device(s) 114, applications 116, databases 118, data storage 120 and server(s) 122. The cloud computing environment 103 may be deployed as public cloud 124, private cloud 126 or hybrid cloud 128. A private cloud 124 may be owned by an organization and only the members of the organization with proper access can use the private cloud 126, rendering the data in the private cloud at least confidential. In contrast, data stored in a public cloud 126 may be open to anyone over the internet. The hybrid cloud 128 may be a combination of both private and public clouds 124, 126 and may allow to keep some of the data confidential while other data may be publicly available.

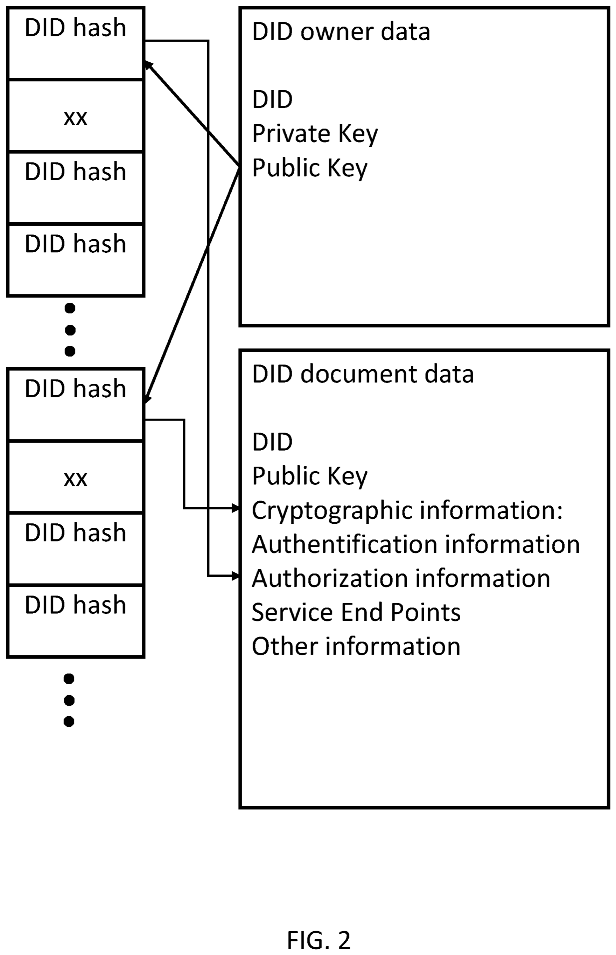

Figure 2 shows an example of ID-based owner data, ID-based passport data and a decentralized identity manager.

The ID may be a decentralized ID (DID). The ID-based passport data may be a DID document associated with the DID. The ID-based owner data may include an ID associated with a subject such as product data or chemical product data or emission data and may include authentication mechanisms. The ID-based owner data may include owner data that is electronically owned and controlled by the DID owner. In this context electronically owned may refer to data that is stored in an owner repository or wallet. Such data may be securely stored and/or managed on an organizational server or client device. The ID-based owner data may include a DID, a private key and a public key. The ID-based owner may own and control the DID that represents an identity associated with the DID subject, a private key and public key pair that are associated with the DID. DID may be understood as an identifier and authentication information associated with or uniquely linked to the identifier.

The DID subject may be a raw material, a basic substance, a chemical product, an intermediate product, a component, a component assembly or an end product. The DID subject may be a machine, a system, or a device used for producing the raw material, the basic substance, the chemical product, the intermediate product, the component, the component assembly or the end product, or a collection of such machine(s), device(s) and/or system(s). The DID owner may be a supply chain participant or a manufacturer such as a chemical manufacturer producing chemicals. The DID owner may be an upstream participant in the supply chain of the chemical manufacturer such as a supplier that supplies raw chemical products or precursors to produce chemicals. The DID owner may be a downstream participant in the supply chain of the chemical manufacturer such as a customer that consumes chemicals to produce the intermediate product, the component, the component assembly or the end product. The DID owner may be any participant of the supply chain including raw chemical product supplier, intermediate chemical products manufacturer, intermediate part manufacturer, component manufacturer, component assembly manufacturer or end product manufacturer.

The DID may be any identifier that is associated with the DID subject and/or the DID owner. Preferably, the identifier is unique to the DID subject and/or DID owner. The identifier may be unique at least within the scope in which the DID is anticipated to be in use. The identifier may be a locally or globally unique identifier for the raw material, the basic substance, the chemical product, the intermediate product, the component, the component assembly, the end product or a collection thereof; the machine, the system, or the device used for producing the raw material, the basic substance, the chemical product, the intermediate product, the component, the component assembly or the end product, or the collection of such machine(s), device(s) and/or system(s); the chemical manufacturer producing chemicals, the upstream participant in the supply chain of the chemical manufacturer, the downstream participant in the supply chain of the chemical manufacturer or a collection thereof; any participant of the supply chain including raw chemical product supplier, intermediate chemical products manufacturer, intermediate part manufacturer, component manufacturer, component assembly manufacturer or end product manufacturer or a collection thereof.

The DID may be a Uniform Resource Identifier (URI) such as a Uniform Resource Locator (URL). The DID may be an Internationalized Resource Identifier (IRI). The DID may be a random string of numbers and letters for increased security. In one embodiment, the DID may be a string of 128 letters and numbers e.g. according to the scheme did:method name: method specific-did such as did:example:ebfeb1f712ebc6f1c276e12ec21. The DID may be decentralized independent of a centralized, third party management system and under the control of the DID owner.

The DID document 16 may be associated with the DID. Accordingly, the DID document 16 may include a reference to the DID, which may be associated with the DID subject that is described by the DID document. The DID document 16 may wincludean authentication information such as the public key. The public key may be used by third-party entities that are given permission by the DID owner/subject 14 to access information and data owned by the DID owner/subject 14. The public key may be used for verifying that the DID owner 14, in fact, owns or controls the DID. The DID document 16 may include authentication information, authorization information e.g. to authorize third party entities to read the DID document or some part of the DID document 16 e.g. without giving the third party the right to prove ownership of the DID.

The DID document 16 may include one or more representations that digitally link to the product data or chemical product data, e.g. by way of service endpoints. A service endpoint may include a network address at which a service operates on behalf of the DID owner. In particular, the service endpoints may refer to services of the DID owner that give access to product data or chemical product data. Such services may include services to read or analyze product data or chemical product data. Chemical product data may include chemical product declaration data, chemical product safety data, certificate of analysis data, emission data, product carbon footprint data, product environmental footprint data, chemical product specification data, product information, technical application data, production data or combinations thereof.

The DID document 16 may include various other information such metadata specifying when the DID document 16 was created, when it was last modified and/or when it expires.

The DID and DID document may be associated with a data registry node such as a centralized data service system or a decentralized data service system, e.g. a distributed ledger or blockchain 10. Possible blockchain systems include Quorum, Hyperledger Fabric or the like. The distributed ledger or blockchain 10 may be used to store a representation of the DID 14 that points to the DID document 16. A representation of the DID may be stored on distributed computing nodes of the distributed ledger or blockchain 10. For example, DID hash may be stored on multiple computing nodes of the distributed ledger 10 and point to the location of the DID document 16. In some embodiments, the DID document 16 may be stored on the distributed ledger. Alternatively, in other embodiments the DID document 16 may be stored in a data storage (not illustrated) that is associated with the distributed ledger or blockchain 10.

The distributed ledger or blockchain 10 may be any decentralized, distributed network that includes various computing nodes that are in communication with each other. For example, the distributed ledger 10 may include a first distributed computing node 12.1, a second distributed computing node 12.2, a third distributed computing node 12.3, and any number of additional distributed computing node as illustrated by 12.4, 12.5. The distributed ledger or blockchain 10 may operate according to any known standards or methods for distributed ledgers. Examples of conventional distributed ledgers that correspond to the distributed ledger or blockchain 10 include, but are not limited to, Bitcoin [BTC], Ethereum, and Litecoin.

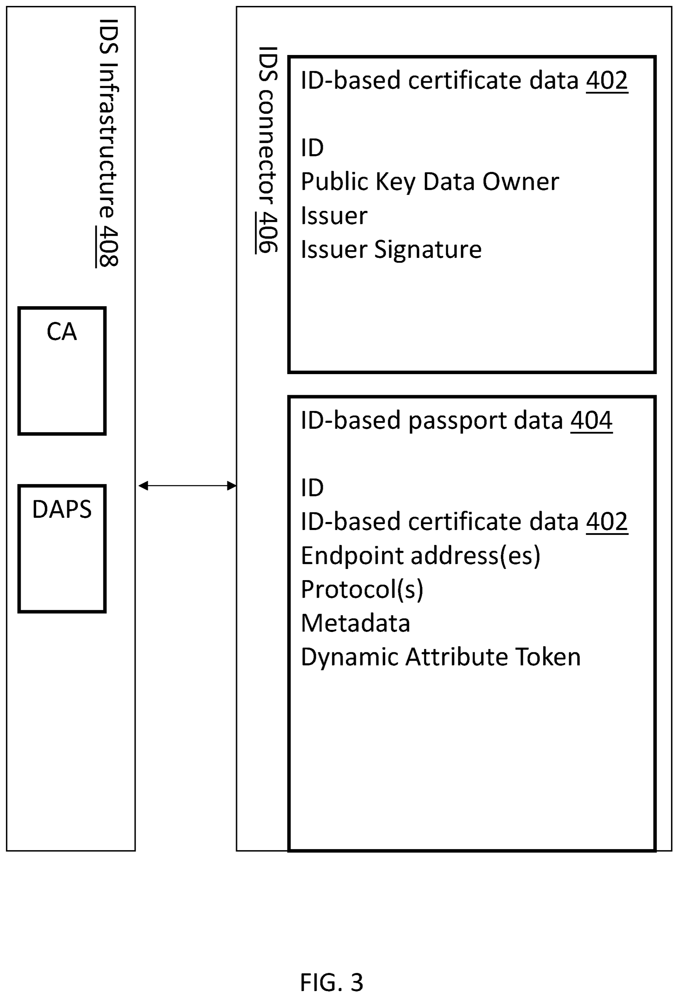

Figure 3 shows an example of ID-based certificate data, ID-based passport data and an identity manager.

In contrast to the example of Fig. 2, the example of Fig. 3 is certificate based. ID-based certificate data may include authentication data of the certificate owner and the certificate issuer. For example, a cryptographic signature from the issuer may bind the public key of the data owner to the ID. The ID may be a unique ID (such as UID) as described in relation to the DID of Figure 2. The certificate may be a X.509 certificate such as X509v3. The ID-based passport data may be associated with the data source of the data owner. The ID-based passport data may include an ID, authentication data and endpoints associated with product data or chemical product data, in particular emission data,. Such endpoints may include any digital representation connecting to the data source. The data source may provide product data and/or chemical product data, in particular emission data.

In this certificate-based example, the ID-based passport data may include one or more certificate(s) associated with the data owner. The certificates may be associated with an identity manager including e.g. a certificate issuing service and/or a dynamic provisioning service providing dynamic attribute tokens (e.g. OAuth Access Tokens). The information required to verify the certificates may be provided via an authentication registry associated with the certificate issuing service and/or a dynamic provisioning service. For instance, in the IDSA Reference Architecture Model, Version 3.0 of April 2019, a connector associated with the data owner, a Certification Authority (CA), a Dynamic Attribute Provisioning Service (DAPS) and a connector associated with the data consuming service are used to verify the identity prior to performing a data exchange (not shown). For this purpose, such connectors may include one or more certificate(s) such as X.509 certificate(s). This way the connector possesses a unique identifier embedded in a X.509 certificate that identifies the connector instance.

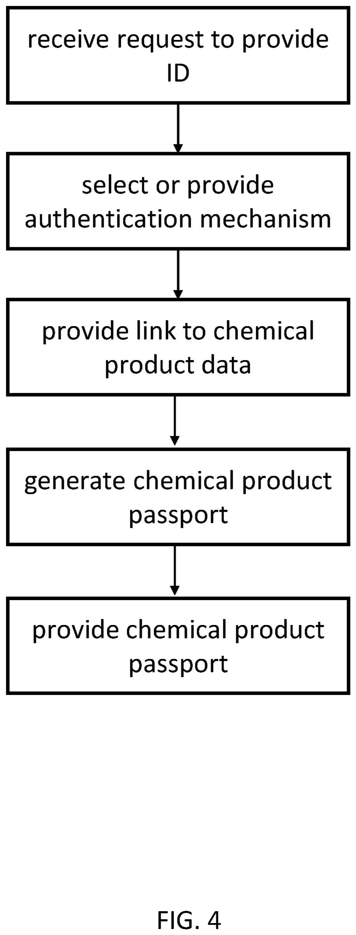

Figure 4 shows an example method for generation of the chemical product passport.

For chemical product passport generation, a request to provide a decentral identifier associated with a data owner and chemical product data, in particular emission data, may be provided. A computing node (that acts as a DID owner's management module, user agent, ID hub and/or certification issuer) may receive an indication to generate a decentral identifier. The indication may include providing at least one authentication mechanism or selecting at least one of multiple authentication mechanisms.

In response to the request, the chemical product passport including the decentral identifier and data related to the chemical product data, in particular emission data, may be generated. An authentication mechanism may be selected or provided. The decentral identifier and data related to the authentication mechanism may be generated or provided. The digital representation connected to the chemical product data, in particular emission data, may be provided. Based on the decentral identifier, data related to the authentication mechanism and the digital representation connected to the chemical product data, in particular emission data, the chemical product passport may be generated.

The chemical product passport may be provided for access by a data consuming service controlled by a data providing service associated with a data owner. The chemical product passport may include at least (1) data related to the decentral identifier and (2) data related to at least one authentication mechanism. Next, at least a portion of data contained in the chemical product passport may be propagated to a authentication data registry such as a distributed ledger. The chemical product passport may further include data related to the chemical product data, in particular emission data.

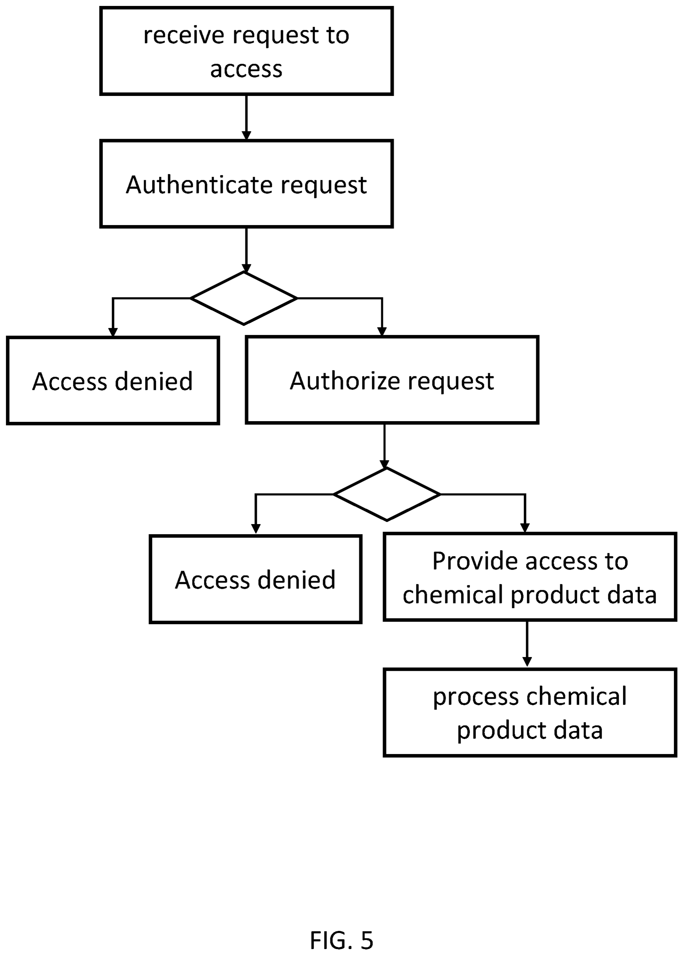

Figure 5 shows an example method for using the chemical product passport to further process the chemical product associated with the chemical product passport.

For using the chemical product passport, an indication to access the chemical product data, in particular emission data, associated with a decentral identifier of the chemical product passport may be received. The chemical product passport may be structured as lined out in Figs. 2 and 3. The chemical product passport may be generated as lined out in Fig. 5.

Before access may be provided to the chemical product data, in particular emission data, the request may be authenticated. In particular, the data consuming service requesting to access the chemical process data and/or the data providing service providing access to the chemical process data may be authenticating.

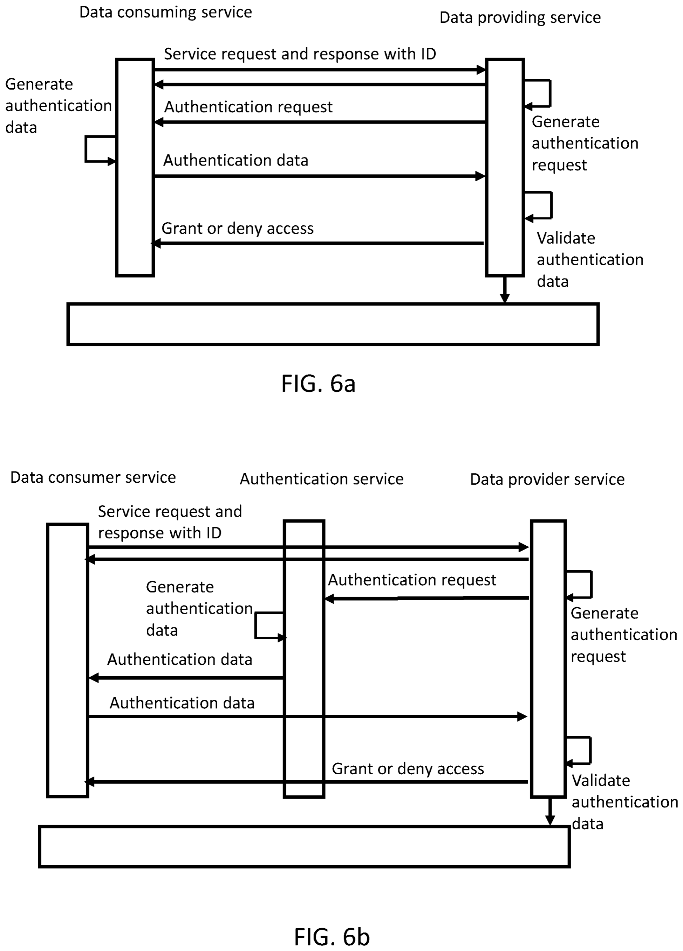

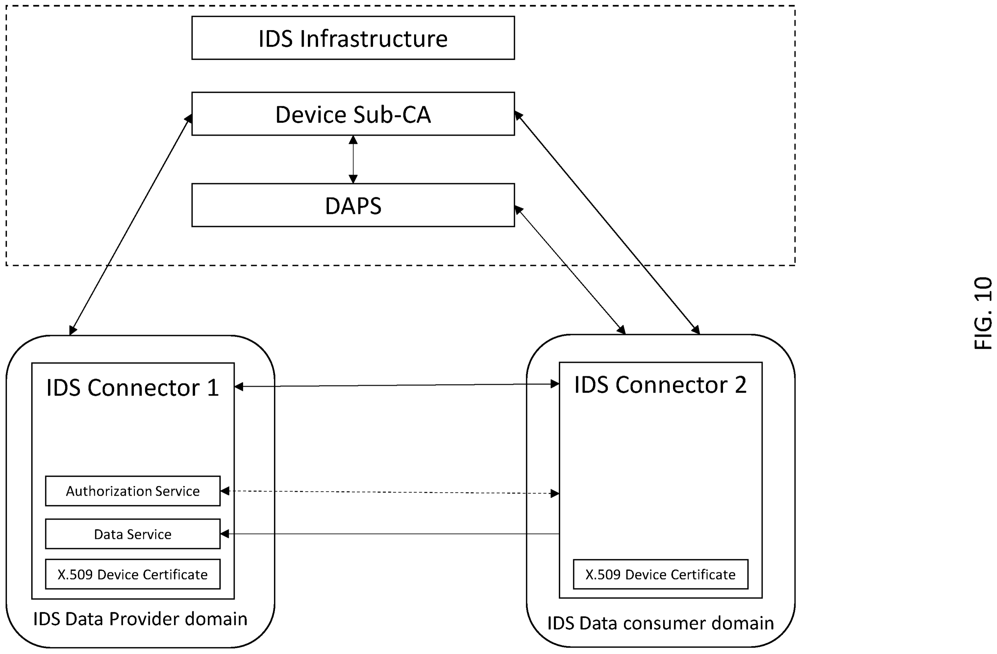

Such authentication may be based on the decentral identity and the data related to the authentication mechanism. The authentication may be performed through different communication patterns, which will be lined out in more detail in Figs. 6 and 10.

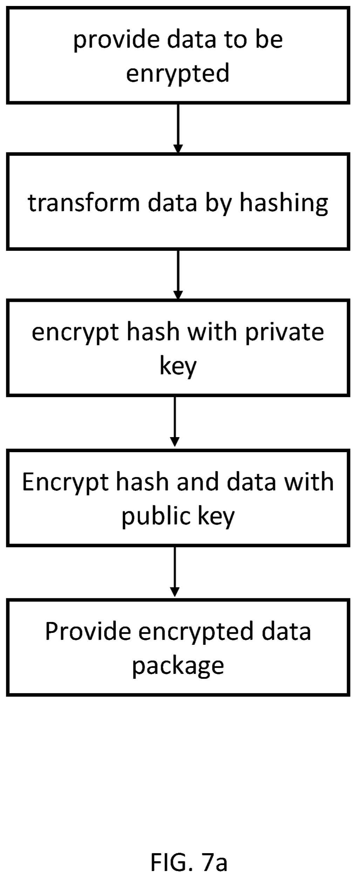

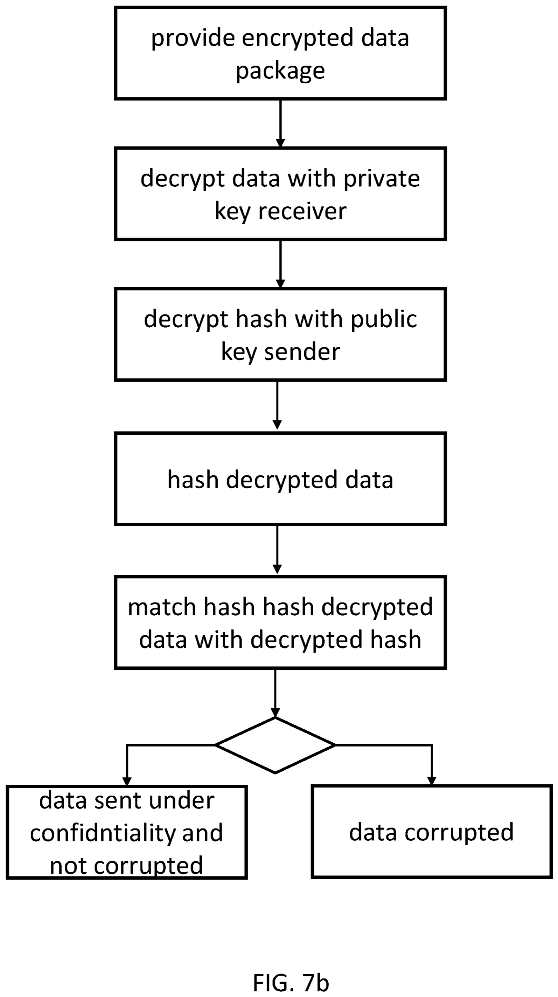

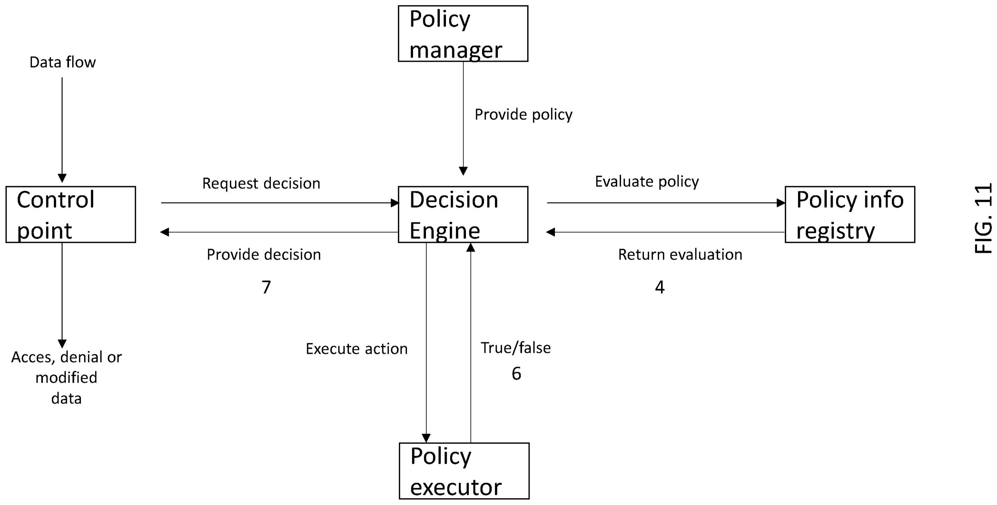

If the authentication fails, access to the chemical product data, in particular emission data, may be denied. If the authentication is valid, an authorization step may follow. Such authorization may be based on the decentral identity and the data related to the authorization rules. The authorization may be performed through different communication patterns, which will be lined out in more detail in Figs. 7 and 11.

If the authorization fails, access to the chemical product data, in particular emission data, may be denied or access may be adapted. In particular, the authorization as requested may be adapted to be in line with the applicable authorization rules. If the authorization is valid, access to the chemical product data, in particular emission data, may be granted according to the authorization rules as requested. Such access to chemical product data, in particular emission data, associated with the decentral identifier may be provided using the representation embodied in the chemical product passport.

Figure 6 shows an example method for authentication to access the chemical product data, in particular emission data.

In the process of authentication, various communication patterns may be implemented to verify identities. Fig. 6a illustrates one example communication pattern that may occur between a data providing service and a data consuming service. In this case, the data providing service may act as verifying entity and no separate service may be used for authentication.

The data consuming service may request a service from the data providing service. The request may include the data consuming service decentral identifier such as a DID.

In response to the request, the data providing service may access a registry such as a central or decentral authentication registry to retrieve data related to the authentication mechanism(s) associated with the ID. For instance, the central authentication registry may provide data related to authentication mechanism via an authentication service issuing access token. Further for instance, the decentral authentication registry may provide data related to authentication mechanism by generating a request token. Data related to authentication mechanism may include a public key of the data consuming service.

Based on the retrieved data related to the authentication mechanism(s), the data providing service may generate an authentication request (corresponding for example to authentication request tokens or dynamic attribute tokens). The authentication request may be generated based on a public key of the data consuming service and/or the private key of the data providing service. The generated authentication request may be sent to the data consuming service.

Based on the received authentication request, the data consuming service may generate authentication data for responding to the authentication request. The generated authentication data may be sent back to the data providing service.

Receiving the response including the authentication data from data consuming service, the data providing service may then validate the authentication data. In response to the validation, the data providing service may grant or deny the service request of the data consuming service.

Fig. 6b illustrates yet another communication pattern that can occur amongst data providing service, an authentication service, and data consuming service.

First, the data consuming service may request a service or initiates a communication with data providing service. The request may include the decentral identifier such as a DID of the data consuming service.

Receiving the request, the data providing service may access a distributed ledger retrieve one or more authentication mechanism(s) associated with the ID. Based on the retrieved authentication mechanisms(s), the service provider may generate an authentication request.

Here, the at least one of the retrieved authentication mechanism(s) may be provided via the authentication service. As such, in some embodiments, the generated authentication request may be sent to the authentication service directly. Receiving the authentication request from the data providing service, the authentication service may generate the authentication data.

The authentication data generated by the authentication service may be sent to the data consuming service.

Data consuming service then, in turn, may pass on the authentication data to the data providing service. Receiving the authentication data, the data providing service may then validate the authentication data. In response to the validation, the data providing service may grant or deny the service request of the data consuming service.

Alternatively, in some embodiments, after the data providing service may generate an authentication request, the data providing service may send the authentication requests to data consuming service. The data consuming service may pass on the authentication request to the authentication service.

Further, after the authentication service may generate the authentication data, in some embodiments, the authentication service merely contacts the data consuming service to notify the receipt of the authentication request and to obtain consent. When the data consuming service receives the notification, the data consuming service may consent and send the consent back to the authentication service. Receiving the consent, the authentication service may then send the authentication data directly to the data providing service.

Finally, in many transactions, the authentication may be mutually performed by both parties. In such a mutual authentication situation, each involved party may be both a subject entity and a verifying entity. Data consuming service and data providing service may have control over their ID. At the beginning, services exchange their ID. Next, each of the services may access a distributed ledger to obtain each other's authentication mechanism(s). Each service may then generate its own authentication request based on the other ID's authentication method(s). The generated authentication data may then be sent to the other service. Receiving each other's authentication data, each service may validate the received authentication data. Based on the validation results, the services may then perform additional communications, e.g. one service may grant or deny the service request of the other service.

Figs. 6a and 6b only show examples of authentication protocols. Also, although the communication arrows were discussed in a certain order or illustrated in a sequence of communications, no particular ordering is required unless specifically stated, or required because a communication is dependent on another communication being completed prior to the communication being transmitted.

Figs. 7a and 7b illustrate the principle of cryptographic signature as it may be used for example in authentication mechanisms based on a private and public key pair. Such processes include cryptographic mechanisms using the key pair and/or hashing functions.

Fig. 7a illustrates one example of an encryption process. In a first step the subject data such as a JWT request token may be provided. Such data may include the public key of the receiver.

Further the private key of the sender may be provided.

In a second step the data or parts of the data may be transformed through a hash function. Hash functions may map multiple inputs to exactly one output. Known hash functions are for instance SHA256, MD5, Bcyrpt or RIPEMD.

In a third step the hashed data or the hash may be encrypted with the private key of the sender. Such encryption of the data or hashed data ensures that the data is provided by the sender as the legitimate owner and not a non-legitimate third-party. This process of encryption with the private key of the sender may also be referred to as signature. The additional use of a hashing function allows to reduce the data package size to be transmitted over the network.

In a fourth step the data and encrypted hash may be encrypted with the public key of the receiver. In a fifth step the encrypted data package including the data and the signature may be provided for transmission to the sender.

Fig. 7b illustrates one example of a decryption process for an encrypted data package of Fig. 7a. In a first step the encrypted data package may be provided. Further the public key of the sender and the private key of the receiver may be provided.

In a second step the data package may be decrypted with the private key of the receiver. This way confidentiality of the data transmission can be ensured.

In a third step the hash of the decrypted data package may be decrypted with the public key of the sender. This way the signature of the sender can be resolved to ensure legitimate sender.

In a fourth step the decrypted data from the decrypted data may be hashed. In a fifth step the hashes from the decrypted hash and the decrypted data may be matched. If the hashes match the data was sent under confidentiality and is not corrupted. If the hashes do not match the data is corrupted.

Figs. 7a and 7b only illustrate the principle of authentication mechanisms based on cryptographic signatures. Multiple different authentication mechanisms exist and multiple variations can be implemented. Further examples of authentication mechanisms include: Biometric authentication like Touch ID or Face ID, FIDO Security keys, push authentication, biometrics and device binding mechanisms, magic links, any combinations thereof or any other password-less authentication mechanism known to the person skilled in the art.

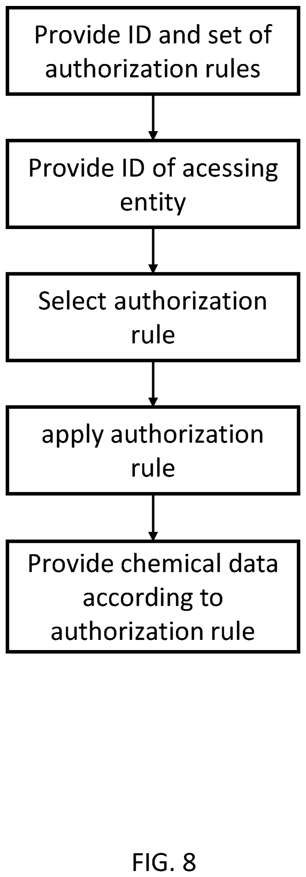

Fig. 8 shows an example method for authorizing access to chemical product data or emission data.

In the following reference to chemical product data may interchangeably used as reference to emission data as one specific embodiment or chemical product data.

In a first step a first decentral identifier and a set of authorization rules for the chemical product data associated with the first decentral identifier may be provided. The set of authorization rules may include usage instructions defining usage policies for entities accessing the chemical product data associated with the decentral identifier. The set of rules may include one or more local rules that are specific to a particular location. The one or more local rules may be based on a location of where the decentral identifier was generated, where the data providing service was implemented, where the data consuming service was implemented or a combination thereof.

The one or more sets of local rules may be based on a location provided by the data providing service or the data providing service. The location may refer to a jurisdiction and the local rules may be associated with legal requirements related to the supply of chemicals or chemical materials. For instance access to chemicals data may be provided via an authorization rule that may include jurisdictional or local rules. Chemicals data may include certificate of analysis data associated with laboratory measurement data acquired from a sample of the raw material, the chemical product, the component, the component assembly, or the end product and/or combinations thereof. The certificate of analysis data may by associated with more than one raw material or chemical product such as those used to manufacture the component. The certificate of analysis data may by associated with more than one raw material or chemical product such as those used to manufacture multiple components to assemble component assembly or end product. chemicals data include chemical product safety data associated with hazards of substances or mixtures of the physical entity of the product such as the raw material, the chemical product, the component, the component assembly, or the end product and/or combinations thereof. The chemical product safety data may by associated with more than one raw material or chemical product such as those used to manufacture the component. The chemical product safety data may by associated with more than one raw material or chemical product such as those used to manufacture multiple components to assemble component assembly or end product. Chemicals data may include chemical product declaration data associated with the physical entity of a product, such as the raw material, the chemical product, the component, the component assembly, or the end product and/or combinations thereof. The chemical product declaration data may be associated with more than one raw material or chemical product such as those used to manufacture the component. The chemical product declaration data may by associated with more than one raw material or chemical product such as those used to manufacture multiple components to assemble component assembly or end product.

In a second step a second decentral identifier or data related to the second decentral identifier of the accessing entity may be provided. Based on the second decentral identifier or data related to the second decentral identifier of the accessing entity, the authorization rule for the chemical product data associated with the first decentral identifier may be selected. The authorization rule may include computer-executable instructions to allow, deny or modify chemical product data. The authorization rule may relate to each data point of the chemical product data or sets or classes of chemical product data. The selected authorization rule may be stored for application to the chemical product data. Such authorization rule may be applied before or on data transaction. The selected authorization rule may be bound to the chemical product data, individual data points or classes of chemical product data for application to the chemical product data. The product data may include data related to different classes of product data, such as chemical product data. For instance, the chemical product data may itself include different classes of chemical product data. In one embodiment at least one class of chemical product data includes chemicals data e.g. data required by regulation or regulatory data for chemicals.

The selected authorization rule may be applied to the chemical product data associated with the first decentral identifier. The selected authorization rule may be applied prior to access of the chemical product data. The selected authorization rule may be applied during run-time on access of the chemical product data.

The chemical product data associated with the first decentral identifier may be provided according to the selected authorization rule. The authorization rule may for instance may include local rules that are specific to a particular location, wherein the location is associated with a jurisdiction and the local rule for the location is associated with legal requirements related to the supply of chemicals. The set of authorization rules may include at least one regulatory instruction configured to provide access to chemicals data relating to regulatory requirements for the supply of chemicals. The provided set of authorization rules may be related to decentral identifiers of accessing entities. the authorization rule may include computer-executable instructions to allow access to chemical product data associated with the first decentral identifier, deny access to chemical product data associated with the first decentral identifier, to modify access to chemical product data associated with the first decentral identifier or to modify chemical product data associated with the first decentral identifier. The authorization rule may relate to each data point of the chemical product data or classes of chemical product data, wherein the selected authorization rule imay be bound to the chemical product data, classes of chemical product data, individual data points or combinations thereof. The set of authorization rules may include one or more of prescribed rules relating to obligations of the data consuming service associated with the second decentral identifier. The set of authorization rules may include one or more of prescribed rules relating to emission data, production data, recyclate content data, bio-based content data, provenance data, labor conditions data or combinations thereof. The set of authorization rules may include one or more processing rules relating to the processing of emission data, production data, recyclate con-tent data, bio-based content data, provenance data, labor conditions data or combinations thereof by a data consuming service associated with decentral identifiers of accessing entities. The set of authorization rules may include one or more aggregation rules relating to bill of material data provided by a data providing service of a supplier or raw material data provided by a data providing service of a supplier.

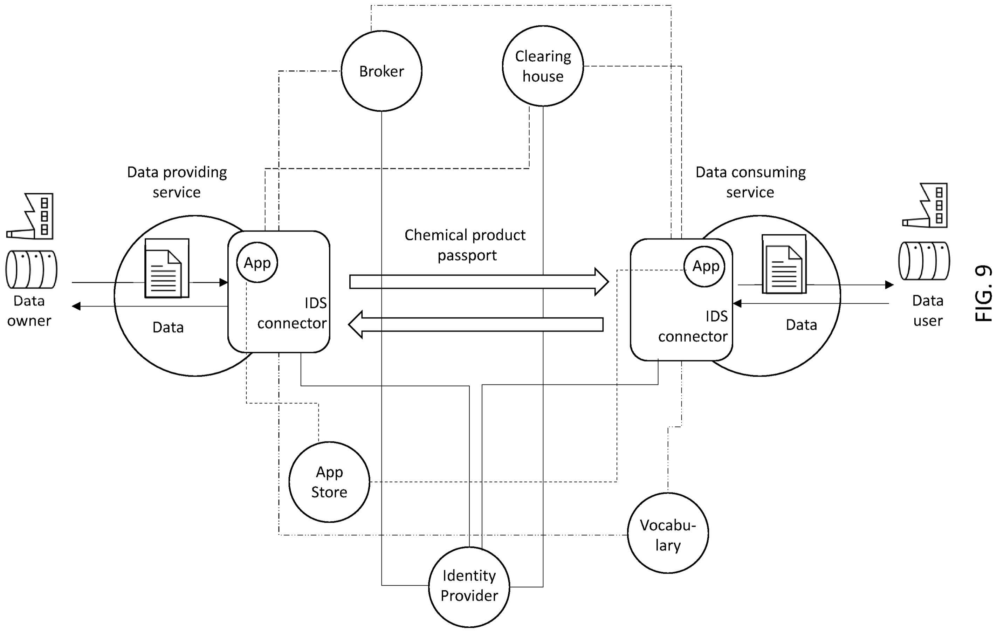

Figure 9 shows a schematic illustration of providing a chemical product passport from a data providing service via the IDS architecture.

In the following reference to chemical product data may interchangeably used as reference to emission data as one specific embodiment or chemical product data.

The data providing service may provide chemical product data to a data consuming service. The following IDS components may be executable components in the "IDS infrastructure" shown in Figure 8: Data Connector, App Store, Identity Provider, Vocabulary Provider, Broker, Clearing House. Data may be exchanged between an interface of a data owner and an interface of a data user via a data providing service and a data consuming service. The data providing service and the data consuming service may each be connected to the data connector to allow a secure and trusted data exchange. The data provided by the interface of the data owner may be associated with authorization mechanisms such as usage policies specifying authorization rules such as data usage rules. Exchange or sharing of data may be conducted according to predefined authorization mechanisms as described in relation to Figure 7. The data provided by the interface of the data owner via the data providing service may be accessed by the interface of the data user according to the usage policies attached to the data provided by the interface of the data owner.

A participant acting as Data Owner may assume the role of the Data Provider. However, there may be cases in which the Data Provider may not coincide with the Data Owner (e.g., if the data is technically managed by a different entity than the Data Owner, such as in the case of a company using an external service to provide data, or if data management activities are handed over to a data trustee). In cases in which the Data Owner does not act as the Data Provider at the same time, the only activity of the Data Owner may be to authorize a Data Provider to make its data available to be used by the interface of the Data Consumer.

The Data providing interface may make data available for being shared or exchanged between a Data Owner and a Data Consumer. To facilitate a data request from a Data Consumer interface, the Data providing interface may provide metadata, such as a description of datasets e.g. the syntax, serialization and/or semantics of data sources, a description of the provider or a description of consumers, to a Broker Service Provider (see below). A Broker Service Provider is not necessarily required for a Data Consumer interface and a Data providing interface to establish a connection.

Exchanging or sharing data with a Data Consumer interface may need not necessarily be the only activity of the Data Provider. The Data providing interface may log the details of the successful (or unsuccessful) completion of transactions at a Clearing House (see below). The data providing interface may facilitate billing or resolve a conflicts. Furthermore, the Data providing interface may use Data Apps to check, enrich or transform the data.

The Data Consuming interface may receive data from a Data providing interface. The Data Consumer interface may the mirror entity of the Data providing interface. The executable components of the Data providing interface may be mirrored by the Data Consumer interface. Before the connection to a Data providing interface can be established, the Data Consumer may search for existing datasets by making an inquiry at a Broker Service Provider. The Broker Service Provider may provide the required metadata for the Data Consumer interface to connect to a Data Provider. Such meta data may include the ID and/or authentication mechanism of the data providing interface. Alternatively or additionally, the Data Consumer interface may establish a connection to a Data providing interface (i.e., with or without involving a Broker Service Provider). In cases in which the information to connect with the Data providing interface may already known to the Data Consumer interface, the Data Consumer interface may request the data (and the corresponding metadata) directly from the Data providing interface. Like a Data providing interface, the Data Consumer interface may log the details of a successful (or unsuccessful) data exchange transaction at a Clearing House, use Data Apps to check, enrich, transform, etc. the data received, or use a Service Provider interface to connect to International Data Spaces (if it does not deploy the technical infrastructure for participation itself).

Similar to the Data Owner being the legal entity that has the legal control over its data, the Data User is the legal entity that has the legal right to use the data of a Data Owner as specified by the usage policy. In most cases, the Data User is identical with the Data Consumer. However, there may be scenarios in which these roles are assumed by different participants.

The identity provider may act as an agent. It may include a Certification Authority (managing digital certificates for the participants of the International Data Spaces), a Dynamic Attribute Provisioning Service (DAPS, managing the dynamic attributes of the participants), and a service named Dynamic Trust Monitoring (DTM, for continuous monitoring of the security and behavior of the network). It may be responsible for issuing technical identities to parties that have been approved to become Participants in the International Data Spaces. The Identity Provider may be instructed to issue identities based on approved roles (see above). The Identity Provider may also manage the PKI rollout. There are two separate PKI hierarchies: one for software signatures (Software Signing Root CA) and one for the IDS Connectors (Service Root CA). An entity is assigned with either an end certificate or a sub/root-CA certificate. The Identity Provider may act as an authentication and/or authorization service by incorporating the DAPS.

Each IDS Connector may run different services and communicate with other IDS Connectors. Using the PKI, an IDS Connector protects the persistent storage of its services and the communication with other IDS Connectors. In order to verify PKI signatures (e.g., for authentication, authorization or for Data Apps that were downloaded), the IDS Connector stores the trusted root certificates (Service Root CA and Software Signing Root CA) in a way their integrity is preserved.

App stores may provide data apps which can be run inside isolated containers of the IDS connectors. These are applications that can be deployed in IDS Connectors to execute tasks like transformation, aggregation or analytics on the data. App stores can be provided by IDS members and must be separately certified under IDS standards. An App Store has a service sub CA. The International Data Spaces Association signs a certificate signing request (CSR) in order to approve Apps or App Stores. The CSR identifies the App Store and makes it possible to sign the service CSRs from the IDS Connectors requesting apps. The IDS Connector creates a key pair for every App it downloads. The private key protects the App's persistent data. When downloading an App from the App Store, the IDS Connector creates a CSR using the public key. The App Store signs the CSR and issues a certificate. The IDS Connector uses this certificate to make sure that the App it is running and is valid.

Vocabulary providers may manage and offer "vocabularies" (including ontologies, reference data models, metadata elements) which can be used to annotate and describe datasets. Vocabulary providers provide these (domain-specific) vocabularies and their references to the IDS Information Model, which is the basis for the description of data sources.

The Broker may be used as an intermediary that stores and manages information about the data sources available in the International Data Spaces. As the role of the Broker is central but non-exclusive, multiple Brokers may be around at the same time (e.g., for different application domains). An organization offering broker services in the International Data Spaces may assume other intermediary roles at the same time (e.g., Clearing House or Identity Provider, see below). The activities of the Broker may include receiving and providing metadata. The Broker must provide an interface for Data Providing interfaces to send their metadata. The metadata may be stored in an internal repository for being queried by Data Consumers in a structured manner. While the core of the metadata model may be specified by the International Data Spaces, a Broker may extend the metadata model to manage additional metadata elements. After the Broker has provided the Data Consuming interface with the metadata about a certain Data providing interface, its job is done (i.e., it is not involved in the subsequent data exchange process).

The Clearing House may be an intermediary that provides clearing and settlement services for financial and data sharing or exchange transactions. In the International Data Spaces, clearing activities are separated from broker services, since these activities are technically different from maintaining a metadata repository. As already stated above, it might still be possible that the two roles "Clearing House" and "Broker" are assumed by the same organization, as both roles require acting as a trusted intermediary between the Data providing interface and the Data Consumer interface. The clearing house and the broker may include a distributed ledger to record metadata from data services. The Clearing House logs activities performed in the course of a data exchange. After a data exchange, or parts of it, are completed, both the Data providing interface and the Data Consumer interface confirm the data transfer by logging the details of the transaction at the Clearing House. Based on this logging information, the transaction may be checked or billed. The logging information can also be used to resolve conflicts (e.g., to clarify whether a data package has been received by the Data Consumer or not). The Clearing House may provide reports on the performed (logged) transactions for billing, conflict resolution, etc.

Figure 10 shows a schematic illustration of the authentication process between the IDS connector 1 present in an IDS data provider domain and an IDS connector 2 present in an IDS data consumer domain.

In the following reference to chemical product data may interchangeably used as reference to emission data as one specific embodiment or chemical product data.

In a typical scenario Connector 2 is associated with a third party that wants to access to chemical product data associated with connector 1 of the chemical product data owner. To provide data from the IDS connector 1 in the IDS data provider domain to the IDS connector 2 present in an IDS data consumer domain the following authentication protocol may be applied.

Connectors may be associated with a certificate issued by the Device-CA. This certificate may serve as the root of identity. The connector may include multiple authentication mechanisms or identity tokens, e.g. a device certificate (X.509v3), a TLS connection certificate (X.509v3) and a 'Dynamic Attribute Token' (OAuth Access Token).

When data within the data service of IDS connector 1 may be accessed by IDS connector 2, an access token may be presented by IDS connector 2. For this purpose, IDS connector 2 may present its X.509 Device Certificate to the DAPS (Dynamic attribute provisioning service) located in the IDS infrastructure to receive a Dynamic Attribute Token (DAT). The 'Dynamic Attribute Token' may include an OAuth Access Token, signed by the Dynamic Attribute Provisioning Service (DAPS). The DAT may be implemented as JSON Web Tokens (JWT) including the cryptographic signature of the DAPS and/or an indication of verified or non-verfied identity attributes of connector 2. Dynamic in this context refers to a short-lived token that contains attributes that the IDS connector 2 possesses. Such short-lived token may only be valid for a limited number of transactions, e.g. one transaction. The DAPS may verify the Device Certificate with the Device Sub-CA prior to issuing the DAT.

The following steps may be performed by Connector 2 to receive the DAT:
A: call token endpoint DAPS with Client Credentials (X.509 Cert)
B: Issue JWT-1{attribute_list, client_id, aud: idsAS:*}

After receiving the DAT, a TLS tunnel may be established by requesting a TLS connection certificate using the same X.509 Device Certificate. This certificate may be automatically requested by the IDS connector 2 by interacting with a ACME server that may be integrated into the TLS Sub-CA present in the IDS infrastructure (not shown).

In one example, an Identity Token may be requested by Connector 2 using the authorization service of Connector 1. This step may be optional. The Identity Token may be requested, for example, if several Access Tokens (Ats) are used. For requesting the Identity Token, Connector 2 may hand in the DAT at the authorization service of Connector 1 and may request an Identity Token. The authorization service in IDS connector 1 may use a database or rule engine to decide whether the Identity Token is to be provided to Connector 2.

The following steps may be performed by connector 2:
Hand in JWT-1 at connector 1 authentication service,
request JWT-2 {scope: C1/PS}
Use Rule Engine for access decision and issue JWT-2{aud: C1} at connector 1
Provide JWT-2 to connector 2

Connector 1 may provide the Identity Token to Connector 2. Connector 2 may hand in the Identity token and optionally DAT request access to the data within the data service of IDS connector 1 via IDSCP (IDS communication protocol). If no Identity Token needs to be requested, Connector 2 may hand in the DAT to Connector 1 to request access to the data within data service of Connector 1 via IDSCP (IDS communication protocol).

Connector 1 may verify the DAT and provide access to the data.

Fig. 11 shows an example implementation of an authorization mechanism using usage-controlled data flow. Such example may be implemented in the international data space (IDS) framework described in Fig. 8.

After access to the IDS connector associated with the data providing service has been granted according to the procedure described in relation to Fig. 9, authorization based on authorization rule(s) may follow. By way of authentication the decentral identifier or data related to the decentral identifier of the accessing entity is available to connector IDS connector.

Usage controlled data flows are one way to enforce authorization rule(s) or usage policies on data exchange or sharing. Usage control may be achieved by binding usage policies to data being exchanged and by continuously controlling the way how data is processed, aggregated or forwarded to other endpoints.

For enforcing usage policies, data flows may be monitored and potentially intercepted by control points. The intercepted data flows may be passed to a decision engine for requesting permission, denial or modification of the data flow. The decision-making may be based on the evaluation of the usage policy by the decision engine. Data usage transactions may trigger interception and evaluation.

To provide the set of authorization rules for the chemical product data associated with the decentral identifier of the IDS connector, usage policies may be provided by a policy manager to the decision engine. The usage policies may be stored independently from the data. The policy manager may be present within the IDS connectors and/or within the clearing house to administer usage policies.

Based on the decentral identifier or data related to the decentral identifier of the accessing entity, the authorization rule for chemical product data associated with a decentral identifier may be selected for enforcement. Enforcement mechanisms may work differently (e.g., work on different system actions) on different systems or technologies. Abstract policies can have different instantiations. Usage policies may be instantiated on the target system. In another example (not shown), usage policies may adhered to the data (also called sticky policy). Sticky policies are one way to cope with the distribution of the usage restrictions. In this approach, machine-readable usage policies may stick to data when it is exchanged. There exist different realization possibilities. For instance, data may be encrypted and can only be decrypted when the adherence to the usage restrictions are guaranteed.

Usage policies may include additional information provided e.g. by a policy information registry. Additional information may include information about contextual information such as previous data usages or the geographical location of an entity, pre- or post-conditions that have to hold before (e.g. integrity checks) and after (e.g. data item is deleted after usage) the decision-making and on-conditions that have to hold during usage (e.g. only during business hours).

In case the decision depends on additional information, the decision engine or the control point may request additional information from the policy information registry. The policy information registry information may provide the requested additional information. In addition, the policy information registry may be used to get contextual information for or about the intercepted system action (e.g., data flow information, geolocation of the requesting device). For example, the policy information registry may be used to resolve the ID of a supplier to a postal address and the postal address to GPS coordinates.

The policy executor may be used to perform additional actions based on policy rules, such as sending a notification e.g. via email when data is used or writing to a system log. The action may be triggered by sending the instruction related to the desired additional action from the control point to the policy executor e.g. after the control point receives the additional information from the policy information system. The policy executor may perform the instructed action and may confirm that the action succeeded to the decision engine.

After the decision engine receives the confirmation from the policy executor, it may forward its decision to the requesting control point. The decision may comprise allowing the data flow, denying the data flow or modifying the data flow. The decision may be implemented by the control point. The selected authorization rule may be applied to the chemical product data associated with a decentral identifier. The chemical product data associated with the decentral identifier according to the selected authorization rule may be provided.

The usage control enforcement may be implemented in the IDS connectors. At runtime, the usage control enforcement may prevent IDS connectors from treating data in an undesired way, for example by forwarding personal data to public endpoints. Application of the usage policy associated with the usage enforcement control at the data providing connector or at the data consuming connector may depends on the usage policy. At the data providing connector, usage policies may specify how often data can be accessed, at what times (e.g., only within business hours), or that data may be filtered or masked (e.g., anonymized) before leaving the company. The usage policies at the data providing connector may include obligations for the data consumer. The technical enforcement may be handled by the control point or the policy executor depending on the usage restriction. For example, limiting data flowing to a specific target system to ensure the correct usage purpose may be handled by the control point while the deletion of data in storage infrastructure outside the connector may be handled by the policy executor.

The usage control enforcement may be implemented at the storage infrastructure. Storage infrastructure may be any kind of storage to persist data such as a file system or a database. The storage infrastructure may be used without modification but every usage of the data may be handled by the IDS connector. In certain instances this could lead to a bottleneck. Usage control may be implemented by encrypting the data within the IDS connector connected to the storage infrastructure before transferring the data to the storage infrastructure. Using the data may bepossible by using the IDS connector to decrypt the data. Hence, every usage may be controlled by the IDS connector. In such cases, usage restrictions such as data lifetime or time constraints may be enforced by deleting the cryptographic key material. Additionally or alternatively, the storage infrastructure may include a usage control enforcement component that monitors and/or controls the usage of the data.

Usage control enforcement may also be implemented in applications such that the data flow within the applications may be controlled and adhered to the usage policies. Similar to the storage infrastructure, a control point may be integrated into the application that controls the data flows.

The afore-described usage control enforcement in the IDS allows to achieve the following security requirement, which cannot be achieved using traditional access control:
- Secrecy: Classified data must not be forwarded to nodes which do not have the respective clearance,
- Integrity: Critical data must not be modified by untrusted nodes as otherwise their integrity cannot be guaranteed anymore,
- Time to live: A prerequisite for persisting data is that it must be deleted from storage after a given period of time,
- Anonymization by aggregation: Personal data must only be used as aggregates by untrusted parties. A sufficient number of distinct records must be aggregated in order to prevent deanonymization of individual records,
- Anonymization by replacement: Data which allows a personal identification (e.g. faces in camera images) must be replaced by an adequate substitute (e.g. blurred) in order to guarantee that individuals cannot be deanonymized from the data,
- Separation of duty: Two data sets from competitive entities (e.g. two chemical industries) must never be aggregated or processed by the same service,
- Usage scope: Data may only serve as input for data pipes within the connector but must never leave the connector to an external endpoint.

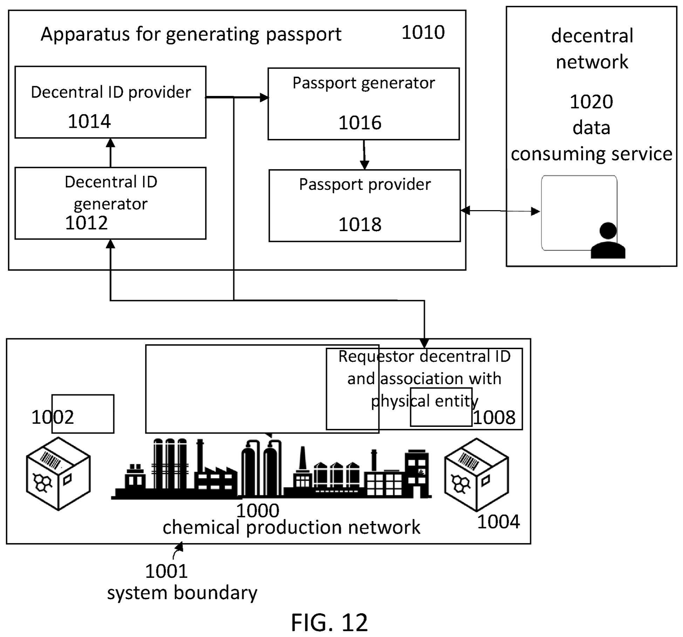

Fig. 12 illustrates an example of an apparatus and associated methods for generating the prod-
uct passport in connection with a chemical product produced by a chemical production network.

The chemical production network 1000 may produce at least one chemical product 1004 from one or more raw material(s) 1002. The raw material(s) 1002 may enter the system boundary 1001 of the chemical production network 1000. The chemical product 1004 may be produced using the raw material(s) 1002. The chemical product 1004 may exit the system boundary 1001 of the chemical production network 1000.

Upon producing the chemical product 1004 or exiting of the chemical product 1004 of the chemical production network 1000, the chemical product passport may be generated. The apparatus 1010 may be configured to generate the chemical product passport. A requestor 1008 may be configured to provide the decentral identifier. The requestor may be configured generate the request for the decentral identifier. Such request may be triggered by a labelling system such as a QR Code generator. The request to provide the decentral identifier may be provided to a decentral identifier generator 1012 configured to generate the decentral identifier. The decentral identifier generator 1012 may provide the decentral identifier to a decentral identifier provider 1014. The decentral identifier provider 1014 may provide the decentral identifier to the requestor 1008 configured to associate the decentral identifier with the chemical product. Such association may include encoding the decentral identifier into a QR code and providing the QR code for labelling the chemical product. This way a physical identifier may be provided that relates the physical entity of the chemical product with the decentral identifier.

The decentral identifier generator 1012 may provide the decentral identifier to a passport generator 1016 configured to generate the chemical product passport. The passport generator 1016 may generate the chemical product passport as described for example in the context of Figs. 2-4. The chemical product passport may include the decentral identifier and data related to the chemical product data. The data related to chemical product data may include one or more digital representation(s) pointing to chemical product data or parts thereof. The chemical product passport may include or be related to one or more authentication mechanisms associated with the decentral identifier and the data related to the chemical product data. The authentication mechanisms may be used as described for example in the context of Figs. 5-11. The chemical product passport may relate to one or more authorization mechanisms associated with the decentral identifier and the data related to chemical product data. The authorization mechanisms may be used as described for example in the context of Figs. 5-11.

The chemical product passport may be provided to a chemical product passport provider 1018. The chemical product passport provider 10018 may be configured to provide the chemical product passport for access by a data consuming service 1020 under control by a data providing service associated with the data owner. The chemical product passport may be used to access chemical product data as for example described in the context of Figs. 5-11.

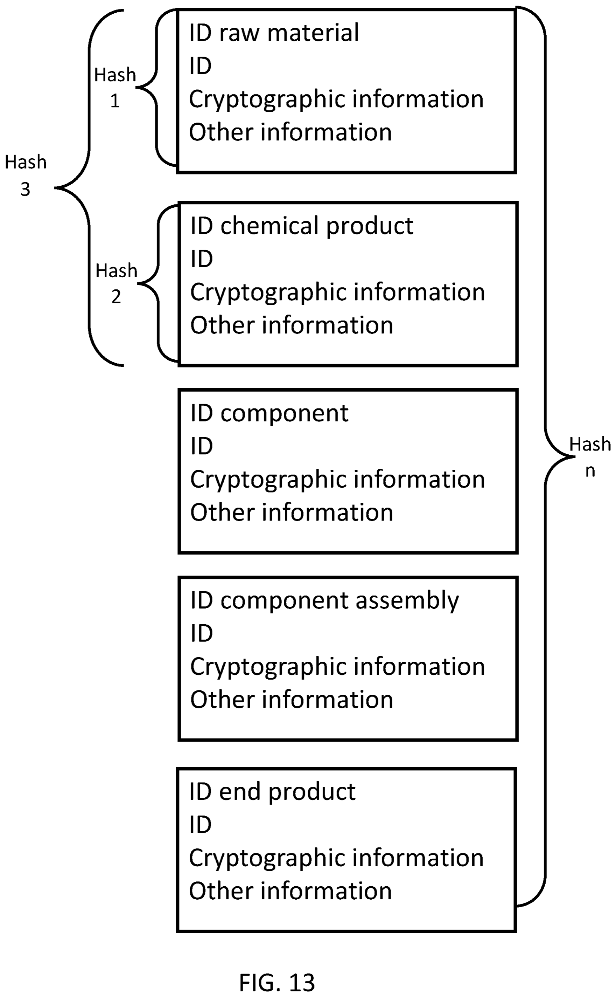

Figs. 13-15 show different example configurations for product passports anchored by digital identifiers. The configurations include different parent, child, grandchild and so on relationships for product passports generated in the chemical value chain up to an end product.

Fig. 13 illustrates an individual configuration for different product passports generated in the chemical value chain. For multiple product stages in the chemical value chain individual product passports may be generated. The product passport generation may include the providing of a decentral identifier and an authentication mechanism for each of the multiple product stages.

The product passports for the multiple product stages may be based on crypto signatures. For instance, the product passports for the multiple product stages may be concatenated through hash values based on different data sets. As shown in Fig. 13 hash 1 may be based on data of the raw material passport, hash 2 may be based on data of the chemical product passport and hash 3 may be based on data of the raw material passport plus data of the chemical product passport. Further concatenation may be done for other combinations of product passports up to hash n, which may concatenate product passports up to the end product passport. Concatenation via hashes of the crypto signature is only one example concatenation. Other examples include permission aggregations with different scope of data that may be embedded in child passports, public key aggregations with different crypto signatures or service endpoint aggregation with different links.

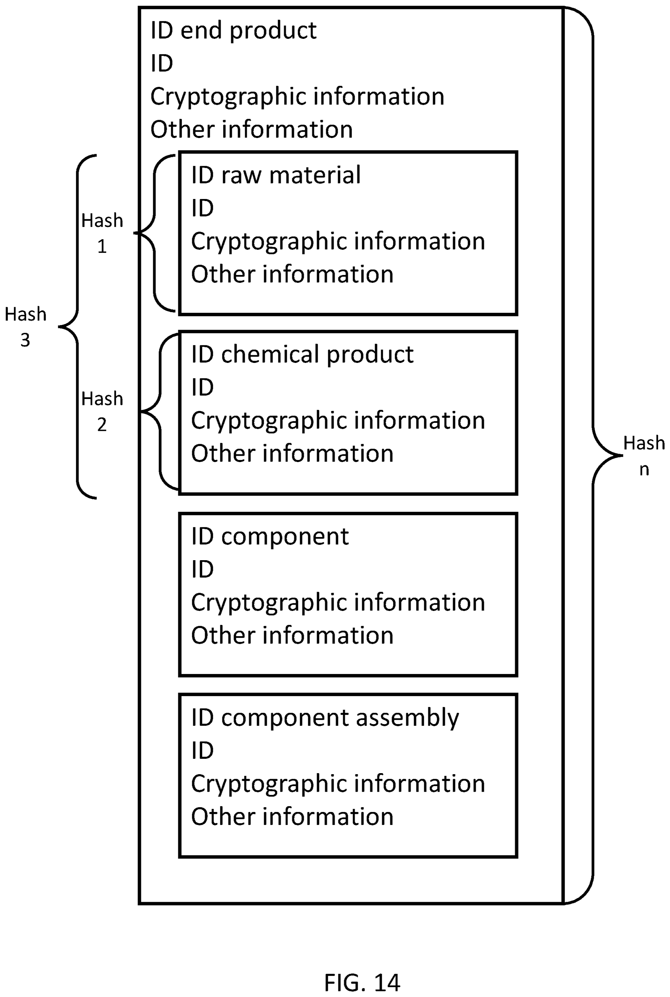

Fig. 14 illustrates an anchored configuration for different product passports generated in the chemical value chain. For the end product an end product passport may be generated. For multiple further product stages in the chemical value chain individual product passports may be generated and embedded in or linked with the end product passport. The product passport generation may include the providing of a decentral identifier and an authentication mechanism for each of the multiple product stages. The product passports for the multiple product stages may be based on crypto signatures. For instance, the product passports for the multiple further product stages may be concatenated through hash values based on different data sets. As shown in Fig. 14 hash 1 may be based on data of the raw material passport, hash 2 may be based on data of the chemical product passport and hash 3 may be based on data of the raw material passport plus data of the chemical product passport. Further concatenation may be done for other combinations of product passports up to hash n, which concatenates product passports up to the end product passport. Concatenation via hashes of the crypto signature is only one example concatenation. Other examples include permission aggregations with different scope of data that may be embedded in child passports, public key aggregations with different crypto signatures or service endpoint aggregation with different links.

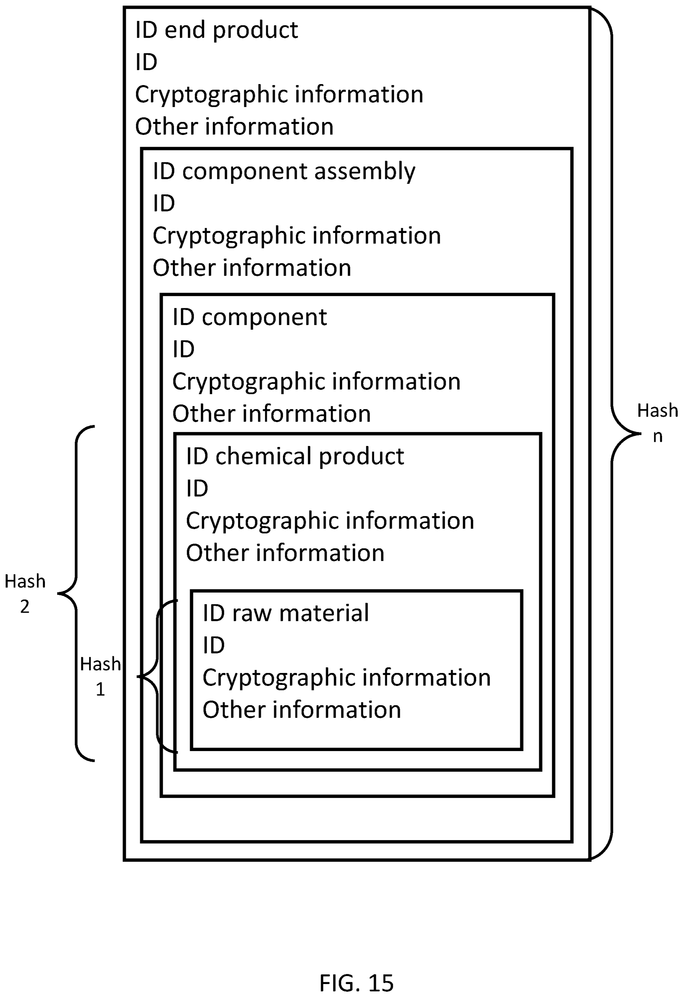

Fig. 15 illustrates a fully embedded configuration for different product passports generated in the chemical value chain. For multiple product stages in the chemical value chain individual product passports may be generated. The product passport generation may include the providing of a decentral identifier and an authentication mechanism for each of the multiple product stages. The product passports for the multiple product stages may be based on crypto signatures. For instance, the product passports for the multiple product stages may be concatenated through hash values based on different data sets. As shown in Fig. 15 hash 1 may be based on data of the raw material passport. Hash 2 may be based on data of the the raw material passport and the chemical product passport. Further concatenation may be done for other combinations of product passports up to hash n, which concatenates product passports up to the end product passport. Concatenation via hashes of the crypto signature is only one example concatenation. Other examples include permission aggregations with different scope of data that may be embedded in child passports, public key aggregations with different crypto signatures or service endpoint aggregation with different links.

The configurations shown in Figs. 13-15 relate to product passports generated in the chemical value chain up to an end product. Similarly, product passports generated in the recycling chain from end product to recyclate(s) may be concatenated. Further similarly, the product passports generated in the chemical value chain up to an end product and in the recycling chain from end product to recyclate(s) may be concatenated. This way the circularity of products and in particular materials may be virtually represented and tracked.

The present disclosure has been described in conjunction with a preferred embodiment as examples as well. However, other variations can be understood and effected by those persons skilled in the art and practicing the claimed invention, from the studies of the drawings, this disclosure and the claims. Notably, in particular, the any steps presented can be performed in any order, i.e. the present invention is not limited to a specific order of these steps. Moreover, it is also not required that the different steps are performed at a certain place or at one node of a distributed system, i.e. each of the steps may be performed at a different nodes using different equipment/data processing units.

In the claims as well as in the description the word "comprising" does not exclude other elements or steps and the indefinite article "a" or "an" does not exclude a plurality. "can" or "may" refers to optional features. A single element or other unit may fulfill the functions of several entities or items recited in the claims. The mere fact that certain measures are recited in the mutual different dependent claims does not indicate that a combination of these measures cannot be used in an advantageous implementation.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "Initiating or causing to perform an action" includes any processing signal that triggers a computing node or device to perform the respective action.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

All terms and definitions used herein are understood broadly and have their general meaning.

## Claims

1. A chemical product passport associated with a physical entity of the chemical product,
wherein the chemical product passport is including the decentral identifier and data related to the emission data, wherein the chemical product passport is associated with a digital representation of the emission data, wherein the digital representation includes a representation for accessing the emission data or part thereof, wherein the decentral identifier is associated with the physical entity the emission data is associated with, wherein via the decentral identifier and its unique association with the data owner and the emission data access to the emission data is controlled by the data owner, wherein the emission data is stored in a data base under control by the data owner, and wherein the chemical product passport is generated and provided according to a method comprising:
receiving a request to provide a decentral identifier associated with the emission data and the data owner,
in response to the request, generating the chemical product passport including the decentral identifier and data related to the emission data, wherein the generation of the chemical product passport includes providing the decentral identifier associated with a physical entity of the chemical product and uniquely associated with the data owner and the emission data;
providing the chemical product passport for access by a data consuming service under control by a data providing service associated with the data owner.

2. The chemical product passport of claim 1, wherein the decentral identifier is provided by one central node or by one or more decentral nodes.

3. The chemical product passport of claims 1 or 2, wherein the decentral identifier is provided to a node generating the chemical product passport and to at least one authentication data registry, preferably accessible by the data providing service and/or the data consuming service.

4. The chemical product passport of any of claims 1 to 3, wherein the chemical product passport includes one or more authentication mechanisms associated with the decentral identifier and the data related to emission data.

5. The chemical product passport of any of claims 1 to 4, wherein the chemical product passport is related to one or more authorization mechanisms associated with the decentral identifier and the data related to emission data.

6. The chemical product passport of any of claims 1 to 5, wherein at least one class of emission data is associated with or includes access restricted emission data associated with the chemical product.

7. The chemical product passport of any of claims 1 to 6, wherein the emission data comprises data related to an environmental footprint, wherein the environmental footprint relates to a product, a company, a process such as a manufacturing process, a raw material, a chemical product, a component, a component assembly, an end product or combinations thereof.

8. The chemical product passport of any of claims 1 to 7, wherein the emission data includes data relating to a carbon footprint of the chemical product.

9. The chemical product passport of any of claims 1 to 8, wherein the chemical product passport is associated with emission data that includes access restricted emission data associated with the physical entity of the chemical product.

10. A computer-implemented method for generating a chemical product passport, the method comprising the steps:
receiving a request to provide a decentral identifier associated with emission data and a data owner,
in response to the request, generating the chemical product passport including the decentral identifier and data related to the emission data, wherein the chemical product passport is associated with a digital representation of the emission data, wherein the digital representation includes a representation for accessing the emission data or part thereof,
wherein the generation of the chemical product passport includes providing the decentral identifier associated with a physical entity of the chemical product and uniquely associated with the data owner and the emission data, wherein the decentral identifier is associated with the physical entity the emission data is associated with,
providing the chemical product passport for access by a data consuming service under control by a data providing service associated with the data owner, wherein via the decentral identifier and its unique association with the data owner and the emission data access to the emission data is controlled by the data owner,
wherein the emission data is stored in a data base under control by the data owner.

11. Use of the chemical product passport as generated according to the method of claim 10 or defined in any of claims 1 to 9 to further process the chemical product associated with the chemical product passport, wherein the chemical product passport includes a decentral identifier associated with emission data and data related to the emission data, wherein the chemical product passport is associated with a digital representation of the emission data, wherein the digital representation includes a representation for accessing the emission data or part thereof, wherein the decentral identifier is associated with the chemical product the emission data is associated with.

12. A system for producing or providing a chemical product associated with a chemical product passport, the system comprising:
a chemical production configured to produce the chemical product connected to or comprising a physical identifier,
an apparatus configured to generate the chemical product passport as defined according to any one of claims 1 to 9.

13. A chemical product associated with a chemical product passport, wherein the chemical product passport including a decentral identifier and data related to emission data is generated and the chemical product is produced by the system of claim 12, wherein the chemical product passport is associated with a digital representation of the emission data, wherein the digital representation includes a representation for accessing the emission data or part thereof, wherein the decentral identifier is associated with the chemical product the emission data is associated with.

14. A computer element with instructions, which when executed on one or more computing node(s) cause the one or more computing node(s) to carry out the steps of the method of claim 10.
